(19) Europäisches Patentamt
European Patent Office
Office européen des brevets

(11) **EP 2 263 183 B1**

(12) **EUROPÄISCHE PATENTSCHRIFT**

(45) Veröffentlichungstag und Bekanntmachung des Hinweises auf die Patenterteilung:
**30.04.2014  Patentblatt 2014/18**

(21) Anmeldenummer: **09778014.2**

(22) Anmeldetag: **14.08.2009**

(51) Int Cl.:
***G06F 19/00*** *(2011.01)*

(86) Internationale Anmeldenummer:
**PCT/EP2009/006053**

(87) Internationale Veröffentlichungsnummer:
**WO 2010/018002 (18.02.2010 Gazette 2010/07)**

(54) **AUTOMATISIERTES SYSTEM ZUR AUSWAHL UND VERMITTLUNG VON GELAGERTEN ALLOGENEN BIOLOGISCHEN ZELLEN FÜR TRANSPLANTATION, THERAPIE UND FORSCHUNG**

AUTOMATED SYSTEM FOR THE SELECTION AND CONVEYANCE OF STORED ALLOGENEIC BIOLOGICAL CELLS FOR TRANSPLANT, THERAPY AND RESEARCH

SYSTÈME AUTOMATISÉ DE SÉLECTION ET DE TRANSFERT DE CELLULES BIOLOGIQUES ALLOGÈNES STOCKÉES DANS UN BUT DE TRANSPLANTATION, DE THÉRAPIE ET DE RECHERCHE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **14.08.2008  EP 08075702**
**19.08.2008  US 90079 P**

(43) Veröffentlichungstag der Anmeldung:
**22.12.2010  Patentblatt 2010/51**

(73) Patentinhaber: **Cytolon AG**
**10559 Berlin (DE)**

(72) Erfinder:
• **KLEIN, Thomas**
**14482 Potsdam (DE)**
• **KELLER, Frank**
**10559 Berlin (DE)**

(74) Vertreter: **Buchanan, Luke Noel et al**
**Hertin und Partner**
**Rechts- und Patentanwälte**
**Kurfürstendamm 54/55**
**10707 Berlin (DE)**

(56) Entgegenhaltungen:
**WO-A-2005/097190       WO-A-2007/070280**
**US-A1- 2002 132 343**

**Beschreibung**

Die Erfindung betrifft ein System für die automatische, schnelle und dynamische Vermittlung von biologischen Zellen für die Transplantation, Therapie oder Forschungszwecke zwischen Entnahmezentren oder Banken (Lagerstätten) und Kliniken, Transplantationszentren oder Forschungsreinrichtungen sowie die Überwachung und Unterstützung der Prozesse von der Anforderungsübermittlung, zur Auslieferung eines für die allogene Transplantation geeigneten Zellpräparates, über die Anwendung der vermittelten Präparate bis zur Nachverfolgung der Ergebnisse im Patienten und die Bereitstellung dieser Daten für statistische und andere Zwecke. Das System ist erstmalig in der Lage, online und automatisch vollständige Lösungsvorschläge für spezifisch gelagerte Transplantationen vorzuschlagen.

In den letzten Jahren haben sich verschiedene Verfahren und Methoden herausgebildet, Nabelschnurblut-Präparate (NSB-) zwischen Entnahmezentren oder Nabelschnurblutbanken einerseits und den Kliniken oder Transplantationszentren andererseits zu vermitteln. Alle Verfahren und Methoden haben ihren Ursprung in den Prozessen, die für die Vermittlung von Knochenmark erforderlich sind. Bislang bestehen keine automatisierten Prozesse. Eine Klink, die für einen Patienten/Empfänger ein NSB-Präparat für die Transplantation benötigt, fragt bei Registern an, ob sie einen entsprechend verschiedener biologischer und medizinischer Kennungen passendes NSB-Präparat für ihren Patienten haben. Die registrierten Daten beziehen sich auf den so genannten HLA Match, auf die im Präparat vorhandene Zellzahl und weitere medizinische bzw. biologische Daten (z.B. Blutgruppe).

Kliniken und Transplantationszentren haben so genannte Koordinatoren, die die Auswahl eines NSB-Transplantates anhand der übermittelten Daten durchführen. Diese Koordinatoren schlagen dem behandelnden Arzt eine Auswahl von Präparaten vor. Der Arzt entscheidet, ob und welches Transplantat genutzt wird. Damit die Kliniken die richtige Nabelschnurblut Einheit bestellen können, muss die Klinik für jedes Präparat alle wesentlichen Daten über das betreffende Präparat erfragen. Für die Informationen, die in einem so genannten Unit Report niedergelegt sind, wurden aber bislang weltweit keine Standards definiert. Auch wurden die Korrelation der Daten einzelner Präparate untereinander bislang nicht erfasst. Koordinatoren unterliegen bei der Präparate Auswahl einem iterativen Prozess, der zeitaufwendig und fehlerbehaftet ist.

Dieses Problem wird dadurch verstärkt, dass viele eingelagerte NSB Präparate, die z.B. nach dem HLA-Match passen würden, für die Transplantation zu klein sind, d.h. dass die Zellzahl des Präparates zu gering ist. Die klinische Forschung der letzten fünf Jahre hat bewiesen, dass die Anzahl der im Nabelschnurblut enthaltenen kernhaltigen Zellen (TC) und nachrangig so genannten CD34+ Zellen (haben die Fähigkeit zur Blutbildung) von entscheidender Bedeutung für den Erfolg der Transplantation sind. Die genetische Passgenauigkeit (HLA-Match) kann bei großer Zellzahl wesentlich geringer sein als vergleichsweise der notwendige HLA-Match bei Knochenmark Transplantationen. Die erforderlichen Zellzahlen und ihre Korrelation zu dem HLA-Match sind zwar im Stand der Technik beschrieben, haben aber noch keinen systematischen Einzug in die etablierten Verfahren gefunden. Koordinatoren und Nabelschnurbanken stehen heute vor der Aufgabe, zueinander passende Präparate in Einzelprüfungen zu identifizieren und mit den Patientendaten zu vergleichen. Dies ist umso schwieriger, da die in Frage kommenden Präparate meist über mehrere Banken verteilt sind und mit verschiedene Verfahren und Standards beschrieben sind.

Im Stand der Technik, beispielsweise der US 2002/0132343 A1, wird offenbart, dass für die erfolgreiche Behandlung von (Leukämie- etc.) Patienten mit passenden Nabelschnurblutpräparaten eine hohe Zellzahl und eine hohe / garantierte Produktqualität (incl. FDA etc. Zertifizierung) notwendig ist und diese zusätzlich zur einfachen und direkten Verwendung in der Transplantationsklinik zur Verfügung gestellt werden können. Es wird Gesamtsystem (SCBS) für expandierte und "gematchte" Stammzellen (nicht nur Nabelschnurblut) beschrieben, welches den gesamten Lebens- und Produktionszyklus umfasst (Quellmaterialgewinnung, Produktion, Zertifizierte Qualitätssicherung und Lieferung). Das Gesamtsystem erfüllt die regulatorischen Qualitätsanforderungen und -standards gemäß FDA etc. wie: FACT, CGTP, AAB und aus einem bestehenden (allogenen) Nabelschnurblutvorrat wird ein Präparat ausgewählt, welches zum Patientengewebe passt (Matching). Die US 2002/0132343 A1 beschreibt, dass die bekannten Methoden zur Typisierung (HLA Typisierung von zumindest sechs Loci) der Spender- und Patientenzellen unter Einhaltung der regulatorischen Standards zum Einsatz kommen sollen. Zusätzlich wird beschreiben, dass ein automatisiertes Trackingsystem zur Verfolgung der einzelnen Präparate/Proben sowie zur Nachverfolgung eingesetzt werden kann. Die Spenderzellen (Quellmaterial) stammen aus einer zertifizierten Quelle (Nabelschnurblutbank), die die relevanten Informationen (z.B. TNC, HLA Loci, Anz. CD34+ Zellen) pro Probe entsprechend den Qualitätsstandards erfasst. Nach der US 2002/0132343 A1 wird das Quellmaterial aufbereitet, so dass nur die relevanten Zellen weiterverarbeitet werden und die gewünschten Zellen (z.B. CD34+ Zellen) werden ex vivo expandiert. Die so produzierten Stammzellprodukte werden als "Patient Treatment Kit" zur Verwendung fertiggestellt. Sie beinhalten dabei eine definierte Charakteristik und sind durch den behandelnden Arzt direkt verwendbar. Ein bestelltes SCBS Produkt matcht mindestens 4/6 Antigene bzw. 3/6 Allele mit $2*10^7$ Zellen/kg bei Kindern (<12J) bzw. Patienten mit <50kg Körpergewicht sowie $1*10^7$ Zellen/kg Körpergewicht bei allen anderen Patienten. Die US 2002/0132343 A1 beschreibt als zentrales Element die Fähigkeit der direkten Lieferung der SCBS-Produkte an die Tansplantationszentren, die das Produkt bestellt haben. Hierzu werden die SCBS Produkte in spezielle Container verpackt, die durch Kurierdienste verfrachtet werden. Dabei werden die Qualitätsstandards aufrecht erhalten. Das beschriebene SCBS System und die Methoden betrachten nicht die Problematik der automatisierten Auswahl geeigneter Präparate. Es wird der heutige Stand der Technik zur manuellen Auswahl von Stammzellpräparaten beschrieben (HLA Matching, Zellzahl/Gewichts-Korrelation, etc.). Die US 2002/0132343 A1 zeigt nicht, wie die benötigte Zeit zur Identifikation geeigneten Spendermaterials reduziert werden kann bzw. wie die notwendigen manuellen Schritte zur Auswahl zwischen mehreren potentiell geeigneten Präparaten automatisiert werden können.

Weiterhin offenbart die US 2002/0168639 A1, dass es bedingt durch die begrenzte Leistungsfähigkeit der Analysegeräte schwierig ist eine Gewebeprobe mit einer Vielzahl von Vergleichsproben zu vergleichen. Die US 2002/0168639 A1 beschreibt einen Profilträger, auf dem einerseits ein Probegewebe untergebracht werden kann und andererseits ein Microarray besteht, auf dem unterschiedliche Vergleichsproben zur simultanen Analyse eingesetzt werden können. Die Reaktionsfähigkeit des Testgewebes bzw. der Microarrays-Proben werden in einer Datenbank gespeichert und in Beziehung zu anderen Informationen des Patienten, von dem das Testgewebe stammt, gesetzt (z.B. Alter, Gewicht, Geschlecht, Krankheitsgeschichte). Das in der US 2002/0168639 A1 offenbarte Datenbanksystem ist an ein Informations Management System (IMS) angeschlossen, dass Suchen und Korrelationen durchführen kann. Damit werden Vergleiche und Korrelationen der biologischen Reaktionsfähigkeiten zwischen dem Testgewebe und den Proben des Microarrays erstellt. Hierzu kommen die - Fähigkeiten von State of the Art Business Analytics Produkten zur Datenanalyse und -visualisierung wie bei "Tibco Spotfire" zum Einsatz. Im Rahmen der US 2002/0168639 A1 werden Informationen über Gewebezellen und deren Spender gewonnen und abgespeichert. Diese Informationen werden über Standard Analyseverfahren miteinander verglichen, um Korrelationen etc. für Forschungs- bzw. Diagnosezwecke zu identifizieren. Es wird nicht offenbart, wie die Korrelation im Bezug auf eine konkrete Fragestellung ablaufen soll. Vielmehr wird auf die allgemeinen Möglichkeiten, die dem Stand der Technik entsprechen, verwiesen.

Die GB 2407193 A beschreibt ein System um biologische Zelllinienexperimente mit Bildauswertung automatisiert ablaufen und auswerten zu lassen. Das System besteht einerseits aus einer Einheit, die es ermöglicht neue Experimente zu definieren und automatisiert ablaufen zu lassen, wobei das System dahingehend offen ist, dass beliebige Experimente und Geräte modular registriert und eingesetzt werden können. Die zweite Systemkomponente umfasst die automatisierte Analyse (Bildauswertung) der Experimente - hierbei wird primär auf die Bildauswertung von Assays referenziert, d.h. die Ergebnisse der Assays (der Experimente) werden in das System gespeist und von diesem analysiert. Es können variable/erweiterbare Analysetechniken eingesetzt werden. Das Gesamtsystem steuert selbstständig die Durchführung und die Analyse mehrerer nacheinander folgender Experimente. Der Durchführungsprozess kann hierzu flexibel definiert/angepasst werden. Die Resultate werden in einer Datenbank abgespeichert und die Ergebnisse über flexibel definierbare Berichte dem Benutzer angezeigt. Die GB 2407193 A zeigt, dass gesamte Laborprozesse für Experimente mit Zelllinien automatisiert werden können. Ähnliches ist aus der industriellen Praxis vieler Anwendungsdomänen bekannt. Die GB 2407193 A gibt ein Rahmengerüst für die automatisierte Datenauswertung im Rahmen des Prozesses vor.

Die US 2004/0121369 A1 adressiert das Problem, den flexiblen Einsatz einer Vielzahl von Geräten und Analysemethoden im Rahmen komplexer biologischer Laborexperimente zu automatisieren. Im Rahmen von sich ändernden Laborprozessen Daten zur Auswertung nacheinander durch unterschiedliche Softwareapplikationen zu schleusen oder die Daten parallel zu verarbeiten, ist aufwändig und bedarf der individuellen menschlichen Koordination (manuelle Datenformatierung oder aufwändige individuelle Programmierung). Die US 2004/0121369 A1 stellt ein flexibles Framework zur Automatisierung von Laborexperimenten und deren Auswertung bereit. Das System ermöglicht die flexible Registrierung (Anbindung) von Laborgerätesteuerungen, um diese im Rahmen von individuell und frei zu definierenden Exerimentenabläufen zu verwenden. Ebenso lassen sich Analysegeräte und Analysesoftware flexibel registrieren und in den Gesamtprozess integrieren. Die US 2004/0121369 A1 beschreibt hierfür einen flexiblen Registrierungsmechanismus, der das Problem der unterschiedlichen Schnittstellen und Protokolle der Geräte und Analyseanwendungen löst, so dass diese effizient miteinander gekoppelt werden können. Die Informationen werden in einer Datenbank gespeichert. Die US 2004/0121369 A1 behandelt das Problem der Effizienzsteigerung bei bestimmten Prozessen. Es wird gezeigt, dass eine Aufgabenstellung, die bisher der manuellen Unterstützung durch Fachpersonal bedurfte, gänzlich automatisiert ablaufen kann. Die Problemlösung liegt jedoch im Bereich der effizienten Gerätekopplung und nicht im Bereich der effizienten Auswahl bestimmter Zellpräparate.

Die WO 02/077640 A2 offenbart ein System, große Datenmengen, die im Zusammenhang mit der Analyse von Biomolekülen mit Hilfe von Microarrays entstehen, effizient aufzubereiten und auszuwerten und gleichermaßen den Analyseprozess zu optimieren. Das offenbarte automatisierte System ermöglicht es, große Datenmengen in einer Datenbank mittels Data Mining Verfahren z.B. nach physikalischen Eigenschaften zu gruppieren und diese Ergebnisse durch ein selbstlernendes neuronales Netz zu analysieren. Mittels mathematischer und statistischer Methoden wird es durch das neuronale Netz ermöglicht, automatisiert neue Proben zu generieren, die eine gewünschte Aufgabenstellung erfüllen. Es werden somit mathematische und statistische (selbstlernende) Algorithmen genutzt, um bestimmte Fragestellungen hinsichtlich eines gesuchten Biomoleküls zu beantworten. Die Algorithmen sind jedoch nicht auf andere System übertragbar, sondern können nur in dem genannten System verwendet werden.

Die US 2008/0014174 A1 beschreibt, die methodische Herstellung von Lymphozytenpräparaten sowie deren Lagerung und ein "Kit" zur fertigen Anwendung beim Patienten. Die Lymphozyten stammen von peripherem Blut von Spendern, die zumindest in 4 Loci mit dem Patienten matchen. Bestimmte Tumore, Virusinfektionen und Autoimmunerkrankungen sollen mittel HLA gematchten allogenen aktivierten Lymphozyten behandelt werden. Es werden keine Aussagen über den genauen Auswahlprozess getätigt.

Weiterhin offenbart die DE 600 30 978 T2 ein Verfahrenwelches es ermöglicht, mehrere biologische Proben mittels einer Sensorplattform simultan in einer hochwertigen Art quantitativ zu analysieren. Hierbei werden insbesondere die chemischen und physikalischen Eigenschaften einer zu analysierenden Probe von der Sensorplattform bestimmt und in eine Signalauswertung eingeführt. Das System kann unter anderem zur Bestimmung der HLA Werten von Proben verwendet werden. Ein weiterer Stand der Technik ist aus WO 2 005 097 190 A2 bekannt.

Im Stand der Technik ist nicht beschrieben, wie genau die Auswahl der Präparate erfolgt. Es ist allgemein bekannt, welche Parameter mindestens zur Auswahl geeigneter Präparate herangezogen werden sollten aber nicht ableitbar, wie aus den analysierten Präparaten auf das "beste" Präparat geschlossen werden kann. Des Weiteren ist im Stand der Technik kein Auswahlsystem beschrieben, dass ein geeignetes Präparat auswählt und dem Koordinator das Ergebnis entsprechend präsentiert und ggf. automatisiert ablaufen kann.

Zusätzlich wird in keiner Schrift die Problematik der Mehrfachtransplantation erwähnt. Dies ist eine Lösungsstrategie für den Fall, dass kein geeignet großes Präparat gefunden wird. Dann wird das Suchproblem auf zwei oder mehr Präparate erweitert, die zusammen genügend Zellen beinhalten und zusätzlich sowohl untereinander als auch zum Patienten eine hinreichende Übereinstimmung in den HLA-Werten besitzen. Auch dieses Problem kann mit der vorliegenden Erfindung automatisiert gelöst werden.

Aufgabe der Erfindung war es demgemäß, ein System zur Verfügung zu stellen, das nicht die Nachteile des Stands der Technik aufweist und die Auswahl und Verteilung eines geeigneten Präparates ermöglicht.

Überraschenderweise wird die Aufgabe durch die unabhängigen Ansprüche gelöst. Bevorzugte Ausführungsformen der Erfindung ergeben sich aus den Unteransprüchen.

Es war völlig überraschend, dass ein System für die Vermittlung und Auswahl von biologischen Zellen oder Geweben, insbesondere Nabelschnurblut-Präparaten, für Transplantationen, Therapien und/oder Forschungszwecke zwischen mindestens einem Entnahmezentrum und/oder einer Lagerstätte und mindestens einer Klinik, einem Transplantationszentrum und/oder einer Forschungseinrichtung, wobei das System die folgenden Schritte umfasst:

- Datenaufbereitung,

- Voreinstellung der Suchkriterien,

- Patientenrecherche und/oder

- Bestellabwicklung und Nachverfolgung, wobei

insbesondere die potentiellen Nabelschnur-Präparate nach einem HLA-Match, einem Patientengewicht, einer Anzahl der kernhaltigen Zellen (TNC) und einer Anzahl der hämatopoetischen Zellen (CD34+) geordnet und ausgewählt werden, nicht die Nachteile des Stands der Technik aufweist.

Im Sinne der Erfindung beschreibt ein System eine Gesamtheit von Einzelbestandteilen, die aufeinander bezogen sind und wechselwirken. Vorteilhafterweise kann ein System sowohl Programme und DV-Anlagen (Datenverarbeitungsanlagen) sowie physikalische Elemente wie Transportbehälter, NSB Präparate umfassen.

Die erfindungsgemäße Lehre ist auch eine Kombinationserfindung, bei der die genannten Elemente zur Erreichung eines technischen Gesamterfolges zusammenwirken und ein synergistischer Effekt entsteht, welcher sich in den überraschenden Eigenschaften des Systems zeigt. Das erfindungsgemäße System, vergleicht die eingehenden Patientendaten mittels einer mehrstufigen Verträglichkeitsmatrix und variierenden Ordnungskriterien mit den Daten registrierter Zellpräparate. Vorteilhafterweise erfolgt der Vergleich vollautomatisch, wobei ein behandelnder Arzt vorteilhafterweise online auf die Daten zugreifen kann. Vorteilhafterweise können dem Arzt automatisch Lösungs-Vorschläge unterbreitet werden, welches Einzel-Präparat (Single-Transplant) oder welche zueinander passenden Präparate (Multi-Transplant) für eine Transplantation in Frage kommen. Damit kann der eigentliche Vorteil der "ready to use" gelagerten NSB-Präparate gegenüber der langwierigen vergleichenden Suche durch Koordinatoren grundlegend verändert und wesentlich verbessert werden. Das System ist für alle biologischen, biochemischen oder chemischen Stoffen geeignet, die einer zeitkritischen Vermittlung bei Transplantationen oder anderen (medizinischen) Anwendungen unterliegen.

Hierfür werden vorteilhafterweise Informationen über Patient und Präparat (z.B. HLA Werte oder Gewicht und Zellzahl) durch informationsverarbeitende Systeme miteinander in Korrelation gesetzt und zur Auswertung von Übereinstimmungen genutzt. Die Informationen über die verfügbaren Nabelschnurblutpräparate (NSBP) werden vorteilhafterweise dezentral durch die Blutbanken zur Verfügung gestellt und aktualisiert. Die Informationen über das verfügbare NSBP Inventar laufen beispielsweise in einem Repository (Datenbank) zusammen und werden dort zur Suche bereitgestellt. Zur Effizienzsteigerung und zur Fehlerminimierung können beispielsweise für die behandelnden Ärzte und Kliniken die Suchparameter, welche bei der Gewichtung und automatisierten Auswahl verwendet werden, zentral gespeichert werden. So können zum Beginn einer Suche die Voreingestellten Suchparametersätze abgerufen werden und ggf. von einem Experten modifiziert werden (Expertenmodus). Die Recherche nach passenden NSBP läuft vorteilhafterweise automatisch ab, kann aber auch von einem Fachmann schrittweise ausgeführt bzw. überprüft werden. Zur Vorbereitung einer Bestellabwicklung ist ggf. eine Interaktion mit der Blutbank notwendig, um weiterführende oder fehlende Untersuchungen zu veranlassen. Dies ist bisher ein manueller und zeitaufwändiger Schritt. Vorteilhafterweise unterstützt das System die Abläufe über einen automatisierten Workflow, das heißt ein Arbeitsablauf, der in einer vordefinierten Abfolge von Aktivitäten in einer Organisation abläuft. Der Workflow informiert stets über die anstehenden Aufträge und über den Arbeitsstand einzelner Aufträge, wodurch die Qualität der Resultate verbessert wird, sowie die Abläufe an sich effizienter und schneller ablaufen. Bei der Nachverfolgung der gelieferten und transplantierten Präparate ist das System in der Lage, die medizinisch und pharmakologisch notwendigen Informationen zusammenzutragen. Außerdem kann das System in einer bevorzugten Ausführungsform automatisiert Statistiken über die Abwicklungsgeschwindigkeit und die Geschwindigkeit der Blutbanken, sowie Erfolgsstatistiken in Abhängigkeit von Krankheitstypen und NSBP Parametern erstellen. So erhält der Benutzer des Systems, beispielweise der Koordinator eine übersichtliche Darstellung der Abwicklung und kann anhand dieser ggf. Arbeitsprozesse, beziehungsweise Bestellabwicklungen verbessern, da er nützliche Auswertungen über beispielsweise die Blutbank erhält.

Bevorzugt ist, dass bei der Datenaufbereitung insbesondere alle in dem System registrierten NSB-Präparate, die weltweit in verschiedenen NSB-Banken oder Entnahmezentren lagern, mit einem vorteilhafterweise einheitlichen Datensatz (Unit Report) als Parameter erfasst werden. Die Parameter umfassen:

- Name und Kennung der lagernden NSB-Bank

- Status der lagernden NSB-Bank hinsichtlich internationaler Zertifizierungen (z.B. Fact)

- Abwicklungszuverlässigkeit der NSB-Bank nach Klassifizierung

- Ansprechpartner an der betreffenden Bank mit Kontaktdaten

- Identifikationsnummer des Präparates

- Anamnese der Mutter, des Kindes und der Familie gemäß Anamnesebogen der Geburtsklinik

- Ethnie der Mutter, des Vaters und/oder des Kindes

- Geschlecht des Kindes

- Einlagerungszeitpunkt des Präparates

- Informationen zur Aufarbeitung des Präparates

- Blutgruppe des Präparates

- HLA-Typus des Präparates

- Zellzahl (TNC) des Präparates

- Zellzahl (CD34+) des Präparates

- Virusstatus des Präparates

- Allele Ausprägungen des Präparates

Die kombinierten Parameter werden vorteilhafterweise in das System eingepflegt und ermöglichen überraschenderweise die eindeutige Bezeichnung eines Nabelschnurblutpräparates (NSBP), da aufgrund der eingepflegten Daten, beziehungsweise der Kombination der Parameter, jedes Präparat durch seine spezifischen Eigenschaften oder Parameter definiert wird. Dies wird vorteilhafterweise durch die kombinierte Erfassung der Parameter erreicht. Im Sinne der Erfindung beschreibt ein Parameter eine Kenngröße, das heißt eine charakterisierende Eigenschaft, die als Daten in das System eingefügt werden. Vorteilhafterweise umfassen diese operationelle Informationen (Attribute) von Patienten, Kliniken, Ärzte, Spender, Blutbanken, NSB-Präparate (Laborwerte, physikalische und informationelle Eigenschaften), Bestell- und Abwicklungs-Informationen und steuernde Informationen umfassend Such- / Ausschlusskriterien, Schwellwerte, Gewichtungsfaktoren.

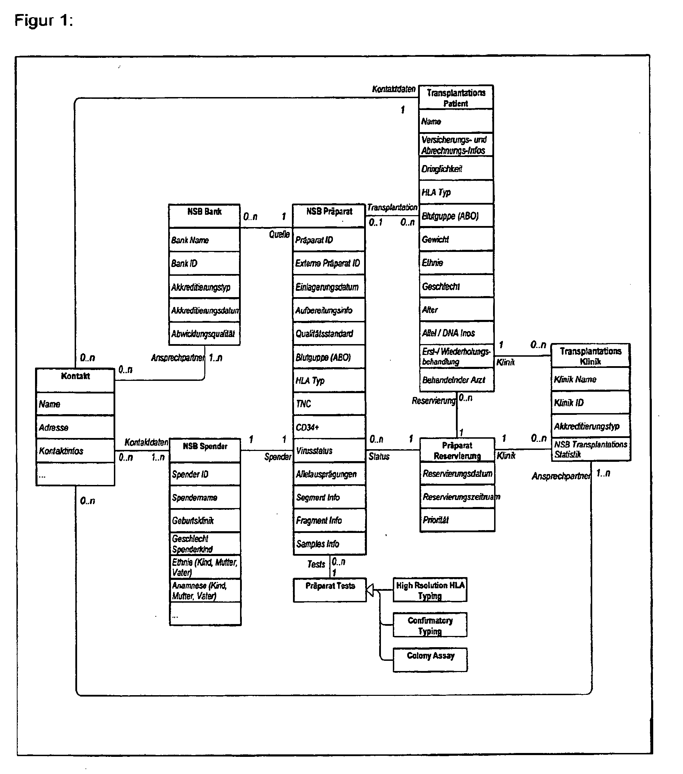

Hierbei werden Parameter erfasst, die zur Kennzeichnung der Präparate verwendet werden. Besonders die vorteilhafte Kombination der Parameter ist nicht im Stand der Technik beschrieben und ermöglicht eine eindeutige Zuordnung und Erfassung eines Präparates. So kann eine Datenbank, im Sinne der Erfindung eine NSBP Datenbank angelegt werden, in die die Parameter gespeichert werden. Ein besonders bevorzugter Aspekt der Ausführungsform ist beispielsweise in Figur 1 gezeigt.

Die Eingabe der Parameter kann beispielsweise dezentral durch die NSB-Bank erfolgen, wobei auch die Datenbank von dieser gepflegt bzw. aktualisiert werden kann. Es werden neben den Informationen der Nabelschnurblut-Bank (NSB-Bank), wie beispielsweise Name und Kennung der NSB-Bank auch der Status der Bank hinsichtlich internationaler Zertifikationen (z.B. Fact - "Foundation for the Accreditation of Cellular Therapy") gespeichert, wodurch sichergestellt werden kann, dass definierte Normen bezüglich der Qualität der Präparate eingehalten werden. Auch ein Ansprechpartner an der betreffenden Bank mit Kontaktdaten kann vorteilhafterweise eingetragen werden. Hierbei umfasst ein Ansprechpartner beispielsweise einen behandelnden Arzt oder einen Koordinator, der an der Bank für die Pflege der Datenbank zuständig ist. Weiterhin wird bevorzugt eine systemeinheitliche Identifikationsnummer (ID) vergeben, die eine eindeutige Zuordnung ermöglicht. Außerdem ist so die Suche nach Präparaten von der NSB-Bank, sowohl übergreifend gegeben. Zusätzlich werden Abwicklungszuverlässigkeitsinformationen für jede NSB-Bank durch das System automatisiert erhoben und bei der Suche berücksichtigt. Des weiteren werden Daten zu der Anamnese der Mutter, des Kindes und der Familie gemäß eines Anamnesebogens der Geburtsklinik in die Datenbank mit aufgenommen. Hierdurch ist vorteilhafterweise eine Beurteilung der Präparate bezüglich bestimmter Krankheiten, beispielsweise Erbkrankheiten möglich. Die Ethnie der Mutter, des Vaters und/oder des Kindes ist als Informationen vorteilhaft, da bestimmte genetische Variationen mit dem ethnischen Hintergrund assoziiert sein können und somit ggf. eine Transplantation verkomplizieren können. Vorteilhafterweise werden weiterhin Parameter wie Blutgruppe, HLA-Typus, Zellzahl (TNC - "total nuclear cells" und CD34+), Virusstatus und allele Ausprägung der Präparate in die Datenbank eingepflegt. Diese umfangreichen Information ermöglichen eine Charakterisierung und Identifikation der Präparate und dementsprechend eine optimale Zuordnung eines Empfängers.

Vorteilhafterweise beinhaltet der Datensatz jeden Präparates Informationen darüber, ob das Präparat in Segmenten (wenn ja, wie viele) und mit Fragmenten (wenn ja, wie viele) und mit DNA-Samples (wenn ja, wie viele) eingefroren wurde. Fragmente, Segmente und Samples dienen der späteren näheren Bestimmung des Präparates hinsichtlich eines bestimmten Patienten und zur Überprüfung zentraler Daten vor der Transplantation. Das System informiert, wie viele der Segmente, Fragmente und DNA-Samples aktuell bei der Anfrage noch vorhanden sind, bzw. welche weiteren Tests wie z.B. CT (Confirmatory Typing) HR (High-Resolution HLA-Typing) oder CA (Colony Assays) bereits durchgeführt wurden bzw. welches die Ergebnisse dieser Test waren. Darüber hinaus wird der Status eines Präparates festgehalten, d.h. ob und seit wann das Präparat möglicherweise von einer Klinik reserviert ist.

Im Sinne der Erfindung kann die Datenbank, umfassend die Daten oder Parameter auch als ein Data Warehouse, d.h. eine zentrale Datensammlung, deren Inhalt sich aus Daten unterschiedlicher Quellen zusammensetzt, bezeichnet werden. Dieses verwaltet nicht nur alle Daten der einzelnen Präparate in den verschiedenen NSB-Banken, sondern es gleicht auch jedes Präparat, das eingestellt wird, dynamisch gegen alle anderen Präparate in den verschiedenen NSB-Banken ab, so dass automatisch mit Registrierung eines jeden Präparates dokumentiert wird, welche Präparate untereinander für eine möglichen späteren Doppel- oder Mehrfachtransplantation (MultiCord) eingesetzt werden können.

Das erste Ordnungskriterium für diesen Multi-Cord-Abgleich zwischen den registrierten Präparaten ist der HLA-Match. In mindestens vier von sechs HLA-Merkmalen herrscht bevorzugt Übereinstimmung, in der Reihenfolge der Eignung als Multi-Cord stehen diejenigen Präparate ganz oben, die die meisten HLA-Matches haben. Präparate, die z.B. einen negativen Virusstatus haben, das heißt einen bestimmten Virus nachweisbar nicht aufweisen, werden nicht berücksichtigt. Im Sinne der Erfindung beschreibt eine Ordnung eine definierte Reihenfolge von Elementen. Die Ordnung der Elemente kann auf deren Eigenschaften, beispielsweise die Parameter oder Attribute (beispielsweise NSB Präparate) bezogen sein. Im Sinne der Erfindung beschreiben die Ordnungskriterien, wie die Ordnung zu Stande kommt (beispielsweise alle NSB-Präparate gemäß Ihrer TNC Größe vom größten zum kleinsten Präparat aufreihen). Vorteilhafterweise können auf eine Ordnung Filterkriterien angewendet werden, das heißt es können beispielsweise nur Präparate für eine Suche berücksicht werden, die eine definierte TNC Größe aufweisen. Besonders vorteilhaft ist, dass bei größeren Datenmengen diese Ordnungen als Index verwendet werden kann, um beispielsweise effiziente Suchen (auch als Kombination über mehrere Kriterien hinweg) durchzuführen.

Das zweite Ordnungskriterium ist die Blutgruppen Gleichheit bzw. Verträglichkeit. Präparate mit Blutgruppen Gleichheit stehen wieder ganz oben, diejenigen mit Verträglichkeit folgen und Blutgruppen die sich ausschließen führen zur Nichteignung als MultiCord hinsichtlich bestimmter anderer Präparate.

Zellzahl (TNC und CD34+), ethnische Zugehörigkeit und Allele-Ausprägungen werden als Informationen oder Merkmale der Präparate mitgeführt und dienen zur Bestimmung der weiteren Reihenfolge, das heißt ob die Präparate geeignet wären und ob es vorteilhaft wäre, ein weiteres Merkmal der Präparate zu überprüfen. Präparate mit hoher TNC-Zellzahl und hoher CD34+ Zellzahl stehen wieder weiter oben. Gleiches gilt für identische ethnische Herkunft und verträgliche Allele Ausprägungen. Damit sind im Datenbestand des Systems, das heißt der in der Datenbank vorliegenden Daten, bereits vor Anfrage durch eine Klinik für einen individuellen Patienten, mögliche Paarungen bzw. Gruppierungen zueinander passender Präparate identifiziert und in Rangfolge gesetzt.

Bevorzugt ist, dass die anfragende Klinik eine Patientenrecherche durchführt, wobei die Ermittlung der zum Patienten kompatiblen Präparate entsprechend folgender Ordnungs- und/oder Ausschlusskriterien umfasst:

-   Name und Kennung der Klinik bzw. des Transplantcenters

-   Name des Koordinators und des behandelnden Arztes mit Kontaktdaten

-   Status der Klinik hinsichtlich internationaler Zertifizierungen (z.B. Fact)

-   Durchschnittliche Anzahl an NSB-Transplantationen an der anfragenden Klinik in den letzten drei Jahren

-   Name des Patienten, Versicherungsnummer und weitere Abrechnungsdaten

-   Anamnese des Patienten

-   Indikation und Therapievorschlag des behandelnden Arztes

-   Dringlichkeit nach definierter Klassifizierung

-   HLA-Typus des Patienten

-   Blutgruppe des Patienten

-   Gewicht des Patienten

-   Ethnie des Patienten

-   Geschlecht des Patienten

-   Alter des Patienten

-   Bekannte Allele Ausprägungen des Patienten und/oder Daten einer DNA-Typisierung

-   Erst- oder Wiederholungsbehandlung

Durch die vorteilhafte Kombination der Ordnungs- und/oder Ausschlusskriterien, die synergistisch zusammenwirken, kann ein Patient eindeutig charakterisiert werden, wobei vorteilhafterweise die Daten eines Patienten mit gespeicherten Daten eines Präparates in einer NSB-Bank verglichen werden. Vorteilhafterweise werden Patient und Präparat mittels den gleichen Kriterien charakterisiert, wodurch ein direkter Vergleich möglich ist. Hierbei können vorteilhafterweise die Eigenschaften der Präparate mit den eines Patienten über beispielsweise eine Verträglichkeitsmatrix in mehreren Stufen und verschiedenen Ordnungskriterien verglichen werden. Die Verträglichkeitsmatrix erlaubt einen direkten und einfachen Vergleich der Eigenschaften der Präparate mit denen des Patienten und gibt Auskunft, ob das Präparat verträglich für den Patienten ist. Vorteilhafterweise werden dem behandelnden Arzt mehrere Ergebnisse, das heißt Präparate präsentiert, die für einen Patienten optimal wären. Des Weiteren können vorteilhafterweise Präparate für SingleCords oder MultiCords hinsichtlich der Transplantation in einen bestimmten Patienten Vorgeschlagen werden. Die finale Entscheidung, welches Präparat oder welche Präparate zur Anwendung kommen, kann vorteilhafterweise durch den behandelnden Arzt getroffen werden. Die Figur 1 zeigt beispielsweise eine besonders bevorzugte Ausführungsform der Ausführungsform.

Im Sinne der Erfindung beschreibt das Kriterium Indikation und Therapievorschlag des behandelnden Arztes, die Diagnose, Analyse und Indikation der Krankheit an der der Patient leidet (beispielsweise Akute Myeloische Leukämie (AML) oder Ischämischer Schlaganfall) und für die der behandelnde Arzt eine bestimmte Behandlung (Therapie) vorschlägt. Der Therapievorschlag umfasst u.a. Festlegung auf das einzusetzende Produkt (beispielsweise Nabelschnurblut-Präparat als Fertigarzneimittel), Zeitpunkt, Ablauf und Dauer der Behandlung sowie Anzahl, Dosierung und Gabe des oder der Produkte und mögliche Maßnahmen für den Fall eines Rückfalls.

Weiterhin beschreibt im Sinne der Erfindung das Kriterium Dringlichkeit nach definierter Klassifizierung, die für alle Nutzer der Datenbank oder Plattform verbindlich festgelegte Priorisierung der Suche und Zuordnung eines geeigneten Präparates für einen bestimmten Patienten gegenüber der parallel laufenden Suche für einen anderen Patienten, die möglicherweise aufgrund ihrer genetischen Typisierungen für das gleiche Produkt im Bestand in Frage kommen. Die Klassifizierungstabelle kann von einem Koordinator festgelegt werden und orientiert sich an der medizinischen Dringlichkeit, mit der der Patient das Präparat benötigt

Vorteilhafterweise können die Parameter, die beispielsweise für einen bestimmten Patienten wichtig sind und die folglich für die Suche nach einem geeigneten Präparat wichtig sind vor der Suche von dem behandelndem Arzt oder der Klinik vordefiniert werden, wodurch eine effiziente und automatisierte Suchabwicklung möglich ist.

Es wird beispielsweise die Information über die behandelnde Klinik nicht nur für die Qualitätssicherung des Prozesses protokolliert, sondern als notwendige Information im Vorfeld erhoben, ohne die der Suchprozess nicht beginnen kann. Weiterhin erhebt die bevorzugte Ausführungsform automatisch eine Statistik pro Klinik über Anzahl und Art der Transplantationen, wodurch die Beurteilung einer Klinik bezüglich ihrer Eignung für eine Transplantation vereinfacht wird. Hierdurch können besonders einfach Kliniken ausgeschlossen werden, die wenig bis keine Erfahrung mit Transplantationen haben.

Die Dringlichkeit des Falles, beispielsweise der Transplantation, wird bei der Priorisierung von Präparaten im Konfliktfall berücksichtigt. Es ist für die Automatisierung der Vorgänge vorteilhaft, wenn potentiell auftretende Konflikte bezüglich der Reservierung und Bestellung von NSBP gelöst werden können. Hierzu kann u.a. die Priorisierungsinformation verwendet werden. Weiterhin werden vorteilhafterweise auch Informationen, die für eine automatisierte Abrechnung notwendig sind, erfasst. Besonders bei der automatisierten Massenabwicklung ist dies eine zwingende Voraussetzung und vereinfacht die Automatisierung massgeblich und ist somit eine wesentliche Reduzierung der Arbeitsschritte.

Während der bevorzugten automatischen Auswahl eines geeigneten Präparates, können vorteilhafterweise auf jeder Stufe Informationen bzw. Listen der geeigneten Präparate abgerufen werden. Hierdurch wird für den Koordinator oder den Fachmann, der die Suche durchführt, die Auswahl übersichtlich gestaltet.

Es ist bevorzugt, dass die Ordnung der potentiellen Nabelschnur-Präparate wie folgt festgelegt ist:

$$ML_{Präp} = \text{Matchlevel entsprechend der HLA Übereinstimmung zwischen Präparat und Patient}$$

$$ML_{Präp} := \begin{cases} 6 : HLA_{Präp} \text{ und } HLA_{Pat} \text{ matchen in } 6 \text{ von } 6 \text{ Werten und Blutgruppenverträglichkeit} \\ 5 : HLA_{Präp} \text{ und } HLA_{Pat} \text{ matchen in } 5 \text{ von } 6 \text{ Werten und Blutgruppenverträglichkeit} \\ 4 : HLA_{Präp} \text{ und } HLA_{Pat} \text{ matchen in } 4 \text{ von } 6 \text{ Werten und Blutgruppenverträglichkeit} \\ Pr\ddot{a}parat \text{ wird nicht berücksichtigt} : sonst \end{cases}$$

$$ZF_{Präp} = \text{der Zellfaktor definiert die benötigten Zellzahl pro kg Patientengewicht bei entsprechndem Matchlevel}$$

$$ZF_{Präp} := \begin{cases} 3 \times 10^7 : ML_{Präp} = 6 \\ 4 \times 10^7 : ML_{Präp} = 5 \\ 5 \times 10^7 : ML_{Präp} = 4 \end{cases}$$

$$OZ_{Präp} = \text{Ordungszahl eines Präparates mit der die Präparate entsprechend TNC und Matchlevel geordnet werden können}$$

$$OZ_{Präp} := \frac{TNC_{Präp}}{ZF_{Präp}}$$

SL$_{Single}$ = Shortlist der zu berücksichtigenden Präparate für Einzeltransplantate

$$SL_{Single} := \left\{ p \in Pr\ddot{a}p \left| \frac{OZ_p}{KG_{Pat}} \geq \frac{1}{kg} \wedge ML_{Pr\ddot{a}p} \geq 4 \right. \right\}$$

Die Standardordnungen der Präparate in einer Shortlist erfolgen nach folgenden Kriterien:

Ordung 1 =   Reihung zuerst nach Matchlevel, dann nach Ordnungszahl, dann nach DC34$^+$

$$\text{Ordnung 1 (SL)} := \left\{ p1 \in SL, p2 \in SL \left| \begin{array}{l} entweder\ ML_{p1} > ML_{p2} \\ oder\ ML_{p1} = ML_{p2} \wedge OZ_{p1} > OZ_{p2} \\ oder\ ML_{p1} = ML_{p2} \wedge OZ_{p1} = OZ_{p2} \wedge CD34_{p1} \geq CD34_{p2} \end{array} \right. \right\}$$

Ordung 2 =   Reihung zuerst nach Ordnungszahl, dann nach Matchlevel, dann nach DC34$^+$

$$\text{Ordnung 2 (SL)} := \left\{ p1 \in SL, p2 \in SL \left| \begin{array}{l} entweder\ OZ_{p1} > OZ_{p2} \\ oder\ OZ_{p1} = OZ_{p2} \wedge ML_{p1} > ML_{p2} \\ oder\ OZ_{p1} = OZ_{p2} \wedge ML_{p1} = ML_{p2} \wedge CD34^+_{p1} \geq CD34^+_{p2} \end{array} \right. \right\}$$

Hierbei entspricht:

Präp          = Nabelschnurblutpräparat

Pat          = Patient

HLA$_{Pat}$          = HLA Werte des Patienten

HLA$_{Präp}$          = HLA Werte eines Präparates

TNC$_{Präp}$          = Anzahl der kernbehafteten Zellen eines Präparates

KG$_{Pat}$          = Körpergewicht des Patienten in kg

CD34+$_{Präp}$          = Anzahl der CD34$^+$ Zellen eines Präparates

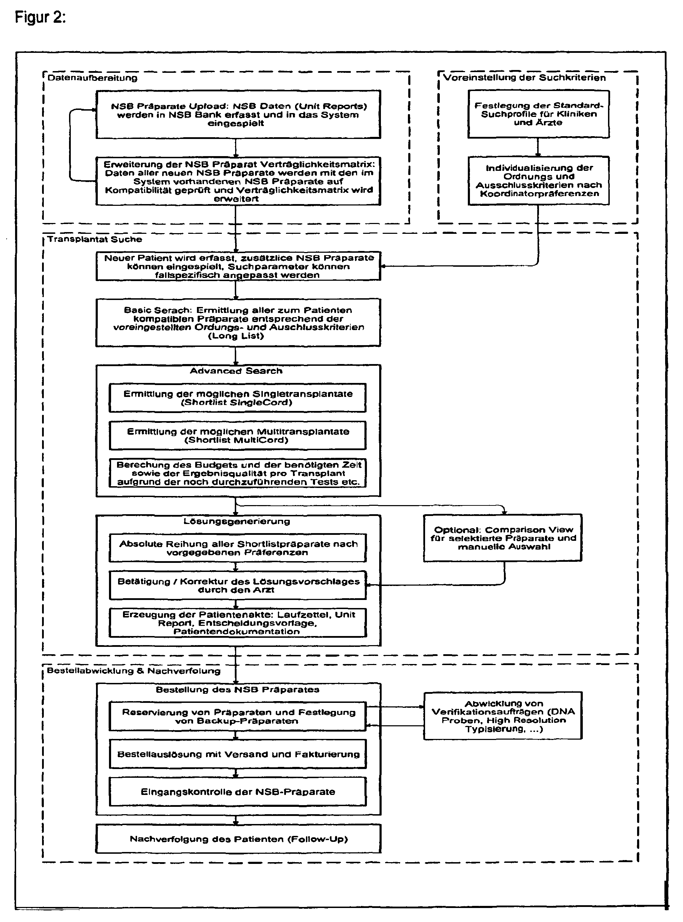

Die bevorzugte Ausführungsform ist demgemäß auch eine Kombination, bei der die genannten Elemente zur Erreichung eines technischen Gesamterfolges zusammenwirken, wodurch ein synergistischer Effekt entsteht, welcher sich in einer überraschend effizienten und schnellen Suche nach adäquaten Präparaten zeigt. Demgemäß können die Ordnungskriterien im Sinne der Erfindung auch als Suchkriterien bezeichnet werden. Vorteilhafterweise kann die Suche automatisch erfolgen, wodurch die Suche erheblich schneller ablaufen kann und keine Fehler durch an der Suche beteiligten Personen erfolgen. Vorteilhafterweise wird durch die Standardisierung des Suchverfahrens und die Kombination der Ordnungskriterien, eine automatisierte Massenabwicklung ermöglicht. Ein besonders bevorzugter Aspekt der Ausführungsform ist beispielsweise in Figur 2 gezeigt.

Die Suche nach einem geeigneten Präparat gliedert sich in mehrere Stufen. Die erste Stufe ist der "Basic Search". Nach einem Ausschlussprinzip erscheinen auf der so genannten "Long List", das heißt eine lange Liste, die vorteilhafterweise alle Präparate in Rangfolge, die in mindestens vier von sechs HLA Typisierungen übereinstimmen umfasst und sich gemäß der Blutgruppenzugehörigkeit nicht ausschließen, d.h. es ergibt sich ein Matchlevel entsprechend der HLA Übereinstimmungen zwischen Präparat und Patient. Die Klinik kann zu diesem Zeitpunkt auch Präparate aus NSB-Banken, die nicht registriert sind, in den "Basic Search" einspielen.

Die nächste Stufe ist der "Advanced Search", wobei vorteilhafterweise eine zweigeteilte "Short List", das heißt eine kurze Liste, genutzt werden kann. Die Liste umfasst vorteilhafterweise mögliche Einzel-Transplantate (SingleCord View). Dies sind Präparate, die in der Korrelation der Ordnungskriterien HLA-Match, Patientengewicht und Anzahl so genannter kernhaltigen Zellen (TNC) sowie Anzahl an hämatopoetischen Zellen (CD34+) als einzelnes Transplantat in Frage kommen. Die Korrelation basiert auf folgenden Kennzahlen. Bei einem HLA-Match von sechs aus sechs benötigt der Patient beispielsweise mindestens $3,0 \times 10^7$ TNC pro kg Körpergewicht des Patienten; d.h. hat der Patient ein Körpergewicht von beispielsweise 55 kg könnte das Präparat insgesamt über mindestens $1,65 \times 10^9$ kernhaltiger Zellen verfügen. Bei einem HLA-Match von fünf aus sechs benötigt der gleiche Patient beispielsweise mindestens $4,0 \times 10^7$ TNC pro kg Körpergewicht, wonach das Präparat beispielsweise bei einem Patientengewicht von 55 kg über mindestens $2,2 \times 10^9$ TNC verfügen könnte. Weiterhin könnte bei einem Match von 4 aus 6 HLA-Typen das Präparat über beispielsweise mindestens $5,0 \times 10^7$ TNC/kg also insgesamt über $2,75 \times 10^9$ TNC verfügen. Somit können vorteilhafterweise Ranglisten der identifizierten Präparate nach beispielsweise zwei wählbaren Kriterien erstellt werden: 1) höchster HLA-Match und nachfolgend höchste relative Zellzahl oder 2) höchste relative Zellzahl und nachfolgend höchster HLA-Match. Falls die ermittelten Präparate die gleiche Positionierung aufweisen, wird die weitergehende Rangfolge der Präparate durch die Höhe der CD34+ Zellzahl festgelegt.

Durch die bevorzugte Ausführungsform und insbesonderen durch die Kombination der Kriterien, die synergistisch zusammenwirken, kann das am besten passende Nabelschnurblutpräparat aus einem gegebenen Vorrat identifiziert werden und zum Versand vorbereitet werden. Vorteilhafterweise erfolgt das Auswahlverfahren des Präparates automatisiert. So ist es möglich, das zeitaufwändige manuelle Auswahlverfahren zu standardisieren und zu beschleunigen, welches heute eine zentrale Schwachstelle in der Lieferkette von Nabelschnurblutpräparaten darstellt.

Bevorzugt ist auch, dass folgende Ordnungskriterien und/oder Ausschlußkriterien herangezogen und individuell gewichtet werden:

- Präparate mit einer CD34+ Zellzahl höher 10% der TNC Zellzahl

- Ausschluß von Präparaten, bei denen im CA (Colony Assay) weniger als 75% der CD34+ Zellen überlebten bzw. aktiviert wurden

- Blutgruppenidentität

- Ethnische Identität

- Geschlecht

- Alter des Präparates

- Akkreditierungsstandard

- Ranking der NSB-Bank

Durch die bevorzugte Ausführungsform kann sichergestellt werden, dass eine optimale Qualität der Präparate garantiert wird und so eine erfolgreiche Transplantation ermöglicht werden kann. Hierfür werden vorteilhafterweise Präparate mit einer CD34+ Zellzahl höher 10% der TNC Zellzahl unterschiedlich gewichtet. Präparate, bei denen im CA (Colony Assay) weniger als 75% der CD34+ Zellen überlebten bzw. aktiviert wurden werden ausgeschlossen, um eine hohe Anzahl an hämatopoetischen Stammzellen zu garantieren. Weitere Kriterien, wie Blutgruppenidentität, ethnische Identität und Geschlecht können weiterhin die Auswahl des Präparates einschränken. Des Weiteren können durch die Bestimmung des Alters des Präparates, alte Präparate ausgeschlossen werden, wodurch vorteilhafterweise nur Präparate zur Transplantation verwendet werden, die ein definiertes Alter nicht überschritten haben, wodurch eine überraschend hohe Qualität sichergestellt wird. Der Akkreditierungsstandard Ranking der NSB-Bank kann ebenfalls zur Auswahl herangezogen werden. Somit können Banken ausgeschlossen werden, die beispielsweise wenig Erfahrung mit der Lagerung oder Transplantation von Nabelschnurblut haben. Durch die Kombination der Ordnungs- und/oder Ausschlußkriterien ist eine qualitative Charakterisierung der Präparate möglich, wodurch eine Abstoßung der Präparate bei einer Transplantation reduziert ist und sichergestellt wird, dass ein Patient "das beste", das heißt das für ihn verträglichste Präparat erhält.

Vorteilhafterweise können Auswahlkriterien festgelegt werden, die die Suche nach einem geeigneten Präparat einfacher gestalten und außerdem die Auswahl eines Präparates vereinfachen können. Hierzu können auch Informationen über die Zuverlässigkeit und der Liefergeschwindigkeit der NSB genutzt werden, die vom System automatisiert erhoben werden.

Diese zusätzlichen Ordnungskriterien können im Rahmen der Klinikpolitik einmalig vergeben oder in jedem Einzelfall neu priorisiert werden. Die Priorisierung entscheidet über die Feinauswahl im finalen Ranking der Präparate für die möglichen Lösungen.

Es ist ebenfalls bevorzugt, dass die bevorzugte Ausführungsform zur Vermittlung von Doppel- oder Mehrfachtransplantationen (Multicord) eingesetzt wird. Hierdurch ist es möglich abhängig von der benötigten Zellzahl, eine Doppel- oder Mehrfachtransplantation durchzuführen. Das heißt, benötigt der Patient mehr Zellen, als durch ein passendes Präparat zur Verfügung gestellt werden können, kann ein weiteres passendes Präparat automatisch gesucht werden.

Bevorzugt ist weiterhin, dass die Auswahl der Multicord-Präparate nach folgenden Ordnungskriterien erfolgt,

$$\text{ML}_{\text{P1P2}} = \text{Verträglichkeit von 2 Präparaten untereinander:}$$

$$\text{ML}_{\text{P1P2}} := \begin{cases} 6 : HLA_{\text{Präp1}} \ und \ HLA_{\text{Präp2}} \ matchen \ in \ 6 \ von \ 6 \ Werten \ und \ Blutgruppenverträglichkeit \\ 5 : HLA_{\text{Präp1}} \ und \ HLA_{\text{Präp2}} \ matchen \ in \ 5 \ von \ 6 \ Werten \ und \ Blutgruppenverträglichkeit \\ 4 : HLA_{\text{Präp1}} \ und \ HLA_{\text{Präp2}} \ matchen \ in \ 4 \ von \ 6 \ Werten \ und \ Blutgruppenverträglichkeit \\ \text{Pr} \ddot{a}parat \ wird \ nicht \ ber\ddot{u}cksichtigt : sonst \end{cases}$$

$$\text{GL}_{\text{Multi}} = \text{Grundliste zur Ermittlung der Auswahlliste für Multipäparate}$$

$$\text{GL}_{\text{Multi}} := \left\{ p \in \text{Pr} \ddot{a}p \left| \frac{OZ_p}{KG_{Pat}} < \frac{1}{kg} \wedge ML_{\text{Präp}} \geq 4 \right. \right\}$$

$$\text{SL}_{\text{Multi}} = \text{Shortlist der zu berücksichtigenden Präparate für Multitransplantate}$$

$$\text{SL}_{\text{Multi}} := \left\{ p1 \in GL_{Multi}, p2 \in GL_{Multi} \left| ML_{p1p2} \geq 4 \wedge \frac{OZ_{p1} + OZ_{p2}}{KG_{Pat}} \geq 1 \right. \right\}$$

Die bevorzugte Ausführungsform bietet einen zweiten Teil der "Short List" den MultiCord View mit Präparaten an, die zueinander gematched, das heißt zueinander passend, sind. Vorteilhafterweise ist bereits die Eignung der verschiedenen Präparate zueinander definiert, wodurch vorteilhafterweise keine Unverträglichkeit zwischen mehreren dem Patienten verabeichten Präparaten entsteht. Vorteilhafterweise sind auf der Short-List Präparate aufgeführt, die bislang noch gar nicht alleine oder in Kombination in Betracht kamen und wodurch die benötigte Zellzahl entweder erreicht oder sogar überschritten wird. Bei MultiCord Präparaten ist dasjenige (Teil-)Präparat als vorteilhaft (als das "erste") anzusehen, dass die höhere CD34+ Zellzahl aufweist. Auf der Short List erscheint zu jedem SingleCord und MultiCord ein vorläufiges Budget, das die Kosten hinsichtlich eines Präparates nach Standardwerten entsprechend dem Status der Präparates kalkuliert.

Die Suchresultate können vorteilhafterweise in einem "Compare View" mit bis zu vier Präparaten, die in übersichtlicher Weise mit den Patientendaten verglichen werden können, dargestellt werden. Der "Compare View" vergleicht alle Daten des Unit Reports mit den Daten des Patienten.

Die letzte Stufe des Auswahlverfahrens sind die Lösungsvorschläge, die vorteilhafterweise einem behandelnden Arzt übersichtlich präsentiert werden. Hierbei umfassen die Vorschläge vorteilhafterweise Einzelpräparate und/oder Multi Cord Präparate. Es ist die abschließende Entscheidung des Arztes, ob und für welches SingleCord oder MultiCord er sich entscheidet.

Für jeden Lösungsvorschlag können vorteilhafterweise Ordner/Akte mit vier Blättern angelegt werden. Diese Akte ist Arbeits- und Kommunikationsinstrument für die Zusammenarbeit des Koordinators mit dem behandelnden Arzt bzw. hinsichtlich des Patienten und er Klinikverwaltung.

Beispielsweise kann dieser Ordner wie folgt aufgebaut sein:

Blatt 1 kann ein Arbeitsblatt oder Laufzettel auf dem das oder die Präparate neben den Patientendaten gezeigt werden und auf der die notwendigen weiteren Schritte, die bis zur Transplantation bearbeitet werden können darstellen. Dies sind vor allem die Anforderung von HR-Typing, DNA-Samples, CAs, aber auch direkte Kontaktaufnahme mit der betreffenden NSB-Bank, Reservierungen bis hin zur verbindlichen Bestellung, Transportlogistik und Fakturierung bis hin zur Übergabe an die Klinik Administration.

Blatt 2 des Lösungsvorschlags kann den oder die vollständigen Unit Reports enthalten.

Blatt 3 des Lösungsvorschlags kann zur Dokumentation der Entscheidung dienen; er fasst die Entscheidungskriterien des Arztes zusammen, stellt das abschließende Budget fest und wird von Arzt abgezeichnet. Auf diesem Blatt kann der Arzt auf andere Lösungsvorschläge verweisen, die alternativ zur Anwendung kommen, wenn sich der gewünschte Lösungsvorschlag aufgrund von Ereignissen nicht oder nicht mehr umsetzen lässt.

Blatt 4 kann eine übersichtliche Darstellung hinsichtlich des Präparates, des Zeitablaufes und der Transplantation im Allgemeinen, das dem Arzt für das Patientengespräch bzw. dem Patienten zur Information zu Verfügung steht umfassen.

Weiterhin können vorteilhafterweise vorgeschlagenen Lösungen im Anhang der Akte dokumentiert werden, sollte sich die erste Lösung nicht Verwirklichen lassen (z.B. Transportschäden oder Verlust eines Präparates aus der ersten Lösung).

Die Blätter aus dem Lösungsvorschlag stehen der Klinik für den weiteren Verlauf wie Abrechnung und Nachverfolgung der Transplantation wie z.B. der Anamnese des Patienten zur Verfügung und werden zu gegebenen Zeitpunkt an die betreffende NSB-Bank bzw. NSB-Banken weitergeleitet.

Während des gesamten Verlaufs bis zur Nachsorge des Patienten, werden vorteilhafterweise Daten von neu registrierten NSB-Präparaten der Klinik automatisch übermittelt und bewertet hinsichtlich des Rankings auf der Long und Short List sowie bei den vorgeschlagenen Lösungen.

Damit ist eine dynamische Verbesserung der Lösungsvorschläge bzw. eine Nachbehandlung des Patienten auf dem jeweils jüngsten Datenbestand gesichert.

In einer bevorzugten Ausführungsform wird ein für die allogene Transplantation geeignetes Zellpräparat ausgewählt. Bei einer Allotransplantation stammt das transplantierte Gewebe nicht vom Empfänger selbst, sondern von einem Spender derselben biologischen Art. Um eine schwerwiegende oder sogar tödliche Abstoßung des Fremdgewebes zu vermeiden, ist für die erfolgreiche allogene Transplantation die möglichst vollständige Übereinstimmung der vom Immunsystem erkannten Merkmale mit dem Empfängergewebe erforderlich. Durch die bevorzugte Ausführungsform kann anhand von den vorgebenden Parameter die Suche nach einem geeigneten, das heißt passenden Präparat einfach, schnell und vorteilhafterweise automatisiert durchgeführt werden, wodurch überraschenderweise die die Gefahr einer Abstoßungsreaktion minimiert ist und einer erfolgreichen Transplantation nichts im Wege steht.

In einer weiteren bevorzugten Ausführungsform erfolgt die automatische und vollständige Auswahl für Singlecord- oder Multicord-Transplantate. Hierbei werden dem behandelnden Arzt und/oder dem Koordinator entsprechende Präparate vorgeschlagen, die basierend auf den Parametern zueinander passen und keine Abstoßungsreaktionen generieren. Es werden vorteilhafterweise die zu einander und zu dem Patienten passenden Präparate entsprechend dargestellt, um so die Auswahl erheblich zu vereinfachen und zu beschleunigen. Der behandelnde Arzt kann dementsprechend beide Auswahlmöglichkeiten dargestellt bekommen und kann selbst beurteilen, ob eine Multicord oder Singlecord Transplantation erfolgen soll. Überraschenderweise können durch eine automatische Auswahl Fehler vermieden werden und Singlecord- und/oder Multicord-Transplantate dem behandelnden Arzt präsentiert werden. Vorteilhafterweise erfolgt die Präsentation in einer übersichtlichen Weise, wodurch die Auswahl der Präparate durch den Arzt vereinfacht wird:

Es ist bevorzugt, dass die Suchkriterien an die eingetragenen Kriterien und/oder Parameter angepasst werden. Bei der Auswahl des geeigneten Präparates wird der aktuelle Typisierungsstatus des NSBP berücksichtigt. Das heißt es wird beispielsweise beurteilt, welche zusätzlichen Untersuchungen usw. notwendig sind, damit das Präparat als geeignet bestätigt werden und transplantiert werden kann. Hierzu nutzt die bevorzugte Ausführungsform automatisiert erhobene Statistiken über die erwarteten Kosten und die benötigte Zeit. Dies ist insbesondere bei zeitkritischen Einsatzszenarien unbedingt notwendig und beschleunigt den Auswahlprozess maßgeblich. Grundsätzlich ist die Erweiterbarkeit des Datenschemas von NSBP und Patient vorteilhaft, um weitere Suchkriterien an den zukünftigen Stand der Technik angleichen zu können.

Weiterhin ist bevorzugt, dass für die Darstellung der über die Suchkriterien erlangten Ergebnisse eine Matrix verwendet wird und die Ergebnisse visuell dargestellt werden. Hierzu bietet die bevorzugte Ausführungsform eine visuelle Orientierung über die besten Suchergebnisse gemäß der aktuell gewählten Suchparameter an. Die Suchergebnisse werden hierzu in einer Matrix angeordnet und visualisiert. Die Matrix kann entsprechend der verschiedenen Kriterien dynamisch umsortiert werden. Die Passgenauigkeit anhand der voreingestellten Suchkriterien wird farbig angezeigt. Im Sinne der Erfindung kann die Matrix als Heat Map bezeichnet werden, bei welcher Daten eines Parameters als Farben in einer zwei-dimensionalen Darstellung aufgeführt werden.

Weiterhin ist bevorzugt, dass für die Beurteilung des Status der Ordnungskriterienermittlung Statistiken über die zu erwartenden Kosten und die zu benötigende Zeit verwendet werden. Vorteilhafterweise können Statistiken für die Suche nach einem geeigneten Präparat für die Ordnungskriterienermittlung herangezogen werden. Hierfür können beispielsweise die zu erwartenden Kosten, die benötigte Zeit, die erfolgreichen Transplantationen einer Klinik und die noch durchzuführenden Untersuchungen in die Auswahl eines Präparates, beziehungsweise in die Ordnung, die ein Präparat einnimmt miteinfließen. Hierdurch können Präparate schneller beurteilt und entsprechend geordnet werden. Außerdem ist eine kosten- und zeitreduzierende Suche möglich.

Somit können automatische und vollständige Lösungsvorschläge für SingleCordoder MultiCord-Transplantate erstellt werden. Der Koordinator und der Arzt können sich vorteilhafterweise auf die Eignung der verschiedenen gut definierten und dokumentierten Lösungsvorschläge konzentrieren. Hierbei ist bevorzugt, dass die Koordination zwischen Klinik, Transplantationszentrum und behandelndem Arzt durch die bevorzugte Ausführungsform erfolgt. So kann sichergestellt werden, dass eine verlässliche Kommunikation zwischen der Klinik, das heißt ggf. dem behandelnden Arzt und dem Transplantationszentrum erfolgt. Die Suchparameter sowie die Ergebnisse werden übersichtlich präsentiert, wodurch die Auswahl erheblich vereinfacht ist. Ebenfalls sind die Parameter, nach derer Basis die Suche erfolgt variabel und können an den Patienten und/oder das gesuchte Präparat angepasst werden. Dies ist ein großer Fortschritt gegenüber der bisherigen Situation, bei der die Koordinatoren gezwungen sind, mögliche Transplantate zu einem sehr frühen Zeitpunkt und nach unterschiedlichen Kriterien zu bewerten. Dies führt heute zu unbefriedigenden Ergebnissen und ist sehr zeit- und personalaufwendig. Somit kann durch die bevorzugte Ausführungsform in einer kurzen Zeit ein oder mehrere passende Präparate gesucht und bestellt werden.

Die Erfindung soll im Folgenden beispielhaft erläutert werden, ohne jedoch auf die Beispiele begrenzt zu sein.

Die Bestellung und Suche nach einem geeigneten Präparat kann aus den folgenden Schritten bestehen:

**1. Neuer Patient**

**1.1 Auswahl des Arztes / der Klinik**

Der Arzt des Patienten stellt einen schriftlichen Auftrag für die Suche eines passenden Nabelschnurblut-Präparats (NSB-Präparat). Gleichzeit erfolgt die Bestätigung der Stammzelltransplantationsindikation, womit eine unberechtigte Suche nach einem NSB-Präparat und somit dessen Blockade für andere Suchvorgänge ausgeschlossen wird. Der Patient wird im System einem Arzt und einer Klinik zugeordnet.

**1.2. Erstellen der Patientenakte**

Mithin wird einer Patientenakte, vorteilhafterweise eine digitale Patientenakte erstellt. Es können folgende Patientendaten in eine Vorlage mit beispielsweise den folgenden Parametern eintragbar sein:

| | | | |
|---|---|---|---|
| Eingabe der Patientendaten: | z. B. | Name: | H. K. |
| Alter: | *1949 | | |
| | | Geschlecht: | männlich |
| | | Körpergewicht | 90 kg |

(fortgesetzt)

| Diagnose: | AML Hochrisikogruppe |
|---|---|
| HLA-Werte: | A*2301, 6801 |
| | B*3501, 4403 |
| | DRB1*1501, 1601 |

Die Vorlagen oder Parameter sind variabel und können vorteilhafterweise einfach um weitere Angaben erweitert werden.

### 1.3. Festlegung eines Suchprofils

Über das Suchprofil wird definiert, welche NSB-Präparate in Frage kommen, z.B.:

- TNC (= Total nucleated Cell Count = Anzahl kernhaltiger Zellen) Mindestmenge an TNC für den Patienten: $3 \times 10^7$/kg des Patienten. (Für Patient H.K. werden bei einem Körpergewicht von 90 kg entsprechend $270 \times 10^7$ TNC im NSB-Präparat benötigt.)

- Anzahl der CD34+ Zellen im NSB-Präparat.

- Übereinstimmung der HLA-Werte von Patient und NSB-Präparat. Die HLA-Werte stimmen vorteilhafterweise in 4 von 6 Parametern überein.

- Vorliegen einer genauen Spezifikation des NSB-Präparats durch die NSB-Bank (Unit Report).

Weitere individuelle Suchparameter können definiert werden. Der Suchkoordinator, der die Suche ausführt, kann aber auch voreingestellte Suchprofile verwenden.

### 2. Suchbeginn

### 2.1. Einzel-Transplantat-Suche

Nach Eingabe der Patientendaten in das System und Festlegung der Suchparameter führt das Programm das Matching durch, das heißt die charakteristischen Eigenschaften eines Präparates werden mit denen des Patienten verglichen. Die Parameter des Suchprofils werden als Filter angewendet. Aus dem Inventar aller gemeldeten NSB-Präparate wird vorteilhafterweise basierend auf der Verträglichkeit, das heißt der Übereinstimmung der Parameter, eine Matchliste für den Patienten H. K. generiert.

### 2.2. Multi-Transplantat Suche

Findet sich kein NSB-Präparat mit einer ausreichenden Zellkonzentration (TNC), das als alleinige Stammzellquelle zur Transplantation verwendet werden kann, so kann vorteilhafterweise nach einem weiteren passenden NSB-Präparat gesucht werden, um genügend Zellen für eine erfolgreiche Transplantation bereitzustellen. Hierfür kann eine Multitransplantat-Suche durchgeführt werden. Die Matchliste ist die Grundlage für diesen weiteren Suchvorgang. Sie kann nach den individuellen Vorgaben des Suchkoordinators sortiert werden. Hierbei kann wieder nach einem NSB-Präparat gesucht werden. Diese sollte vorteilhafterweise in 4 von 6 HLA-Werten sowohl passend zum Patienten als auch passend zum ersten NSB-Präparat sein. Dafür werden neue Matchlisten generiert.

### 3. Lösungsgenerierung

Mit den neuen Matchlisten, können sich für eine Multitransplantat-Suche mehrere Lösungen für den Patienten H. K. ergeben. Dennoch ist für den Patienten H. K. eine Mindestmenge von $270 \times 10^7$ Zellen in den NSB-Präparaten vorteilhaft.

### 3.1. Lösungsbericht / Reservierung

Die verschiedenen möglichen Lösungen werden vom Suchkoordinator bewertet. Die endgültige Auswahl kann dem behandelnden Arzt in Form eines Berichts vorgestellt werden. Nach eingehender Prüfung entscheidet dieser über die Auswahl der einzelnen NSB-Präparate.

Für jedes ausgewählte NSB-Präparat werden Aufträge an die NSB-Bank erteilt:

Das NSB-Präparat könnte für den Patienten reserviert werden, wobei dies vorteilhafterweise von der NSB-Bank bestätigt wird. Ohne Reservierung gibt es keine garantierte Verfügbarkeit des Transplantates. Sollte eine Reservierung nicht möglich sein, kann ein anderes NSB-Präparat ausgewählt werden. Hier beginnt der Suchprozess erneut.

### 3.2. Verifikation

Für alle als Lösung definierten NSB-Präparate kann eine Verifikation durchgeführt werden. Dies umfasst z.B.:

- Für jedes NSB-Präparat kann eine DNA-Probe angefordert werden. Der Versand und Empfang wird über das System abgewickelt und bestätigt.

- Der Suchkoordinator kann eine High Resolution Typisierung bei der NSB-Bank in Auftrag geben. Das Ergebnis dieser Typisierung wird von der NSB-Bank über das System gemeldet.

Dieses System von Auftragserteilung und Empfangsbestätigung stellt sicher, dass alle notwendigen Verifikationen der ausgewählten NSB-Präparate sehr schnell und effizient erfolgen. Die Dauer der Auftragsabwicklung durch die NSB-Banken ist genau festgelegt.

3.3 Nach Übermittlung aller relevanten Daten für das jeweilige NSB-Präparat, erfolgt die endgültige Auswahl der Lösungswege durch den Suchkoordinator in Absprache mit dem behandelnden Arzt.

3.4 Die definierten Lösungen werden vorteilhafterweise nach Qualität der NSB-Präparate in erste und zweite Wahl eingeteilt. Vorteilhafterweise ist eine Backup-Lösung vorhanden, damit zum Zeitpunkt der Transplantation, passende NSB-Präparate zur Verfügung stehen.

### 4. Bestellung von NSB-Präparaten

4.1. Die Anforderung der NSB-Präparate erfolgt, sobald der behandelnde Arzt einen Transplantationstermin festgelegt hat. Die NSB-Bank wird über diesen Termin frühestmöglich informiert, damit entsprechende Vorbereitungen getroffen werden können. Dazu gehört vor allem die Bereitstellung eines Stickstoffbehälters zum Transport der NSB-Präparate. Der eigentliche Transport wird von der NSB-Bank organisiert, da dort die Informationen vorliegen, wann das Transplantat abholbereit ist. Sollte die NSB-Bank feststellen, dass ein Transport nicht möglich ist, weil beispielsweise das NSB-Präparat beschädigt wurde, kann auf die Backup-Lösung zurückgegriffen werden.

4.2. Nach Eingang des NSB-Präparats im Transplantationszentrum, wird der einwandfreie Zustand des Stickstoffbehälters, in dem das Präparat transportiert wurde und des NSB-Präparats überprüft und der NSB-Bank bestätigt werden, z.B. können Unterbrechungen der Kühlkette dramatische Auswirkungen auf die Lebensfähigkeit der Zellen des NSB-Präparats haben.

### 4.3. Eingangskontrolle der NSB-Präparate

Durch das Labor der Transplantationsklinik können noch einmal die HLA-Werte bestimmt, die Zellzahl nach dem Auftauen des NSB-Präparates festgelegt und die Vitalität der Zellen überprüft werden. Nach dieser Eingangskontrolle steht das NSB-Präparat für den Patienten zur Verfügung.

### 5. Transplantation

Es ist vorteilhaft, wenn die Transplantation unmittelbar nach der Eingangskontrolle erfolgt, da die Zellen des NSB-Präparats umgehend verabreicht werden sollten.

Die Abläufe von 4.1 bis 5. können für jedes einzelne NSB-Präparat durchgeführt werden. Speziell bei Multitransplantat-Anforderungen, die aus unterschiedlichen NSB-Banken stammen, ist eine sorgfältige Koordination der Bestellung notwendig, um das zeitgleiche Eintreffen der verschiedenen NSB-Präparate im Transplantationszentrum zu sichern.

**6. Follow-up**

Nach der Transplantation kann eine Erhebung der klinischen Ausgangsdaten des Patienten (z.B. Konditionierungsprotokoll, Grunderkrankung, vorherige Chemotherapieprotokolle) erfolgen. Unmittelbar daran kann sich die Erfassung des Transplantationsverlaufes anschließen, wie beispielsweise:

■ Dauer des Engraftments (Anwachsen des Transplantates).

■ Dauer bis zur Funktionsfähigkeit des Transplantats.

■ Klinische Probleme hervorgerufen durch eine mögliche Abstoßungsreaktion.

Diese ausführlichen klinischen Daten werden vorteilhafterweise auch an die NSB-Bank gemeldet, da solche Informationen für die Qualitätssicherung wichtig sind. Das Follow-up nach einer Transplantation erfolgt in regelmäßigen Intervallen.

Der größte Vorteil von cryokonservierten NSB-Präparaten gegenüber Präparaten von Spendern (Knochenmark, peripheres Blut) liegt in der sofortigen "ready-to-use Verfügbarkeit. Diese sofortige Verfügbarkeit kann in der Praxis nur durch ein System zum Tragen kommen, wie es oben beschrieben wurde.

Die Erfindung wird nunmehr anhand von Figuren beispielhaft erläutert, ohne auf die Beispiele begrenzt zu sein. Es zeigen:

Figur 1    Das Basis-Datenmodell

Figur 2    Den Prozessablauf

Figur 3    Die Systemarchitektur

Fig. 1 zeigt eine beispielhafte Darstellung einer bevorzugten Ausführungsform des Basis-Datenmodell. Bei einem ersten Kontakt, können die Daten einer Person aufgenommen werden, wie beispielsweise Name, Adresse und sonstige Kontaktinfos. Es kann beispielsweise ein NSB Spender sein, wobei hier noch weitere Daten in die Datenbank eingegeben werden können (umfassend Geburtsklinik und Anamnese von der Mutter, Vater und/oder dem Kind). Vorteilhafterweise können auch Daten der NSB-Bank erfasst und gespeichert werden. Vorteilhafte Daten umfassen Abwicklungsqualität und eine spezifische Bank ID. Die Daten der NSB Präparate umfassen vorteilhafterweise HLA Typ, TNC Nummer, oder Virusstatus. Anhand dieser Information kann ein Präparat exakt charakterisiert werden, wobei zusätzliche Information über das Präparat durch weitere Tests (umfassend "High Resolution HLA Typing", hochauflösende HLA Typisierun oder Colony Assays). Das Präparat wir durch die Tests untersucht und die Qualität kann einfach beurteilt werden. Vortelhafterweise werden die Daten de Präparates mit den Daten eines Transplantations Patienten verglichen, das heißt es kann beispielsweise die Blutgruppe und die Dringlichkeit verglichen werden. Basierend auf dem Vergleich kann das Präparat vorteilhafterweise für den Patienten von der Transplantations Klinik reserviert werden. Dies kann beispielsweise von einem Koordinator oder einem behandelnden Arzt der Klinik erfolgen. Vorteilhafterweise können Daten der Klinik aufgenommen und in eine bevorzugte Datenbank gespeichert werden. Hierbei umfassen die Daten Klinik ID oder Akkreditierungstyp.

Figur 2 zeigt eine beispielhafte Darstellung des Prozessablaufes. Bei der Datenaufbereitung können neue NSB Präparate in der NSB-Bank erfasst werden und in das bevorzugte System eingespielt werden. Vorteilhafterweise können die neu erfassten Präparate mit den im System vorhandenen NSB Präparaten auf Kompatibilität verglichen werden. Vorteilhafterweise kann dies beispielsweise über eine Verträglichkeitsmatrix erfolgen. Wenn ein NSB Präparat gesucht wird, können die Standard-Suchprofile für Kliniken und Ärzte verwendet werden, wobei vorteilhafterweise auch eine Individualisierung der Ordnungs- und Ausschlusskriterien nach Koordinationspräferenz möglich sein kann. Die Suche nach einem für einen Transplantations Patienten passenden NSB Präparat kann weiterhin fallspezifisch angepasst werden. Es kann eine sogenannte Basic Search erfolgen, bei der vorteilhafterweise alle der zum Patienten kompatiblen NSB-Präparate entsprechend der voreingestellten Ordnungs- und/oder Ausschlusskriterien gesucht werden (Long List). Des Weiteren kann es vorteilhaft sein, eine sogenannte Advanced Search durchzuführen, die die möglichen Singletransplantate (SingleCord) und/oder Multitransplantate (MultiCord) ermittelt. Außerdem könnte vorteilhafterweise das benötigte Budget, die benötigte Zeit, sowie die Ergebnisqualität pro Transplantat aufgrund der noch durchzuführenden Tests aufgeführt werden. Hierbei können die gefundenen Präparate in einer Vergleichsansicht (Comparison View) miteinander verglichen werden, wobei auch einzelne Präparate miteinander veglichen werden können. Vorteilhafterweise kann eine Lösungsgenerierung erfolgen, die die Shortlistpräparate nach den vorgegebenen Referenzen aneinander reiht. Dem zu behandelnde Arzt kann so eine übersichtliche Darstellung der Präparate präsentiert werden, wobei vorteilhafterweise der generierte Lösungsvorschlag von dem Arzt bestätigt oder korrigiert werden kann. Die so generierte Lösung kann in die Patientenakte des Transplantationspatienten aufgenommen, umfassend die Laufzettel, Unit Report, Entscheidungsvorlage und Patientendokumentation. So können vorteilhafterweise in einer Akte alle für eine NSB Transplantation relevante Informationen gespeichert werden. Sobald die Transplantationsklinik, beziehungsweise der behandelnde Arzt ein oder mehrere NSB-Präparate ausgewählte haben, kann das Präparat bei der NSB-Bank bestellt werden. Vorteilhafterweise können die durch die Suche gefundenen Präparate für einen Patienten oder für eine Klinik reserviert werden, wobei vorteilhafterweise Backup-Präparate festgelegt werden können, falls die gewählten Präparate nicht zur Verfügung stehen. Weiterhin können zusätzliche Verifikationen (umfassend DNA Proben, High Resolution Typisierung) die Qualität und Kompatibilität der Präparate sicherstellten. Nach einer Auswahl, können die Präparate bestellt und geliefert werden. Vorteilhafterweise erfolgt über die Präparate eine Eingangskontrolle bei der Klinik. Nach erfolgreicher Transplantation kann vorteilhafterweise eine Nachverfolgung des Patienten (Follow-up) erfolgen.

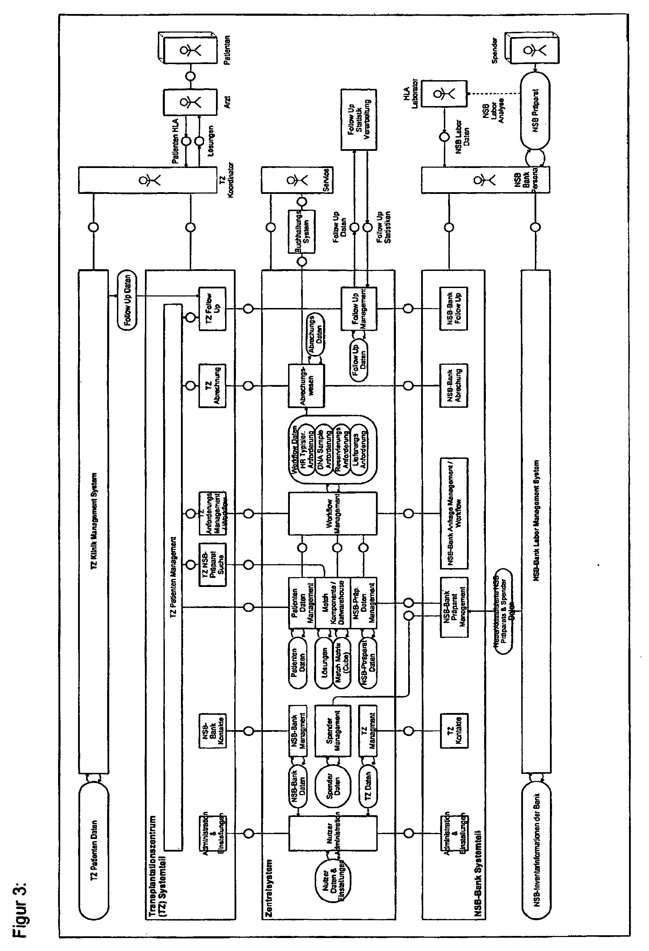

Figur 3 zeigt eine beispielhafte Darstellung der bevorzugten Systemarchitektur.

Die Darstellung zeigt einen schematischen Aufbau eines bevorzugten datenverarbeitenden Systemteils. Die bevorzugte Ausführungsform des Systems kann in 3 Teilbereiche Zentralsystem, TC (Transplantationszentrum) Systemteil und Nabelschnurblutbank (NSB-Bank) Teilsystem gegliedert sein. Das System ist vorteilhafterweise über das Inter- und Intranet nutzbar. Es wird gezeigt, dass NSB-Präparate von Spendern geliefert und durch ein HLA Labor analysiert werden können. Die Präparate können vorteilhafterweise von der NSB-Bank physikalisch aufbereitet und gelagert werden, wobei die gewonnenen Daten über das NSB-Präparat können vorteilhafterweise bei der NSB-Bank in einem Labor Management System verwaltet werden. Informationen ausgewählter NSB-Präparate können beispielsweise als Datensätze dezentral und inkrementell in das Zentralsystem überspielt werden. Der Nabelschnurblutbank bietet das bevorzugte System vorteilhafterweise die Möglichkeit zur Administration, Einsicht von Kontakten der Transplantationszentrum (TZ) Ansprechpartner, Verwaltung der eingespielten NSB-Präparate, komfortable Abarbeitung von Anfragen und Workflow Verfolgung, Verwaltung der kompletten Abrechnung von NSB-Präparat Lieferungen und Dienstleistungen sowie die Verwaltung von Follow-Up Informationen. Weiterhin ist in Figur 3 erkennbar, dass ein Arzt die HLA-Werte von Patienten ermitteln kann und beispielsweise an den TZ Koordinator zur Suche von NSB-Präparaten nebst weiteren Informationen übergeben kann. Der Koordinator kann beispielsweise eine systemgestützte Suche durchführen und liefert Lösungsvorschläge und vorteilhafterweise NSB-Präparate zur Transplantation. Die Patientendaten können vom Transplantationszentrum beziehungsweise der Klinik in einem eigenen Management System verwaltet werden. Vorteilhafterweise können Follow-Up Informationen nach der Transplantation hieraus an das Zentralsystem weitergegeben werden. Dem Transplantationszentrum kann das System beispielsweise die Möglichkeit zur Administration, Einsicht von Kontakten der NSB-Bank Ansprechpartner, Suche nach NSB-Präparaten, komfortable Beauftragung und Kontrolle von Anfragen und Workflow Verfolgung, Verwaltung der kompletten Abrechnung von NSB-Präparat Lieferungen und Dienstleistungen sowie die Verwaltung von Follow-Up Informationen bieten. Das Zentralsystem kann vorteilhafterweise einen gesicherten Zugang zu den Daten, die zur Wahrung der Datensicherheit verschlüsselt abgelegt sind (z.B. in einem Datenbanksystem) ermöglichen. Die Nutzerdaten und deren Einstellungen können vorteilhafterweie zentral abgelegt werden, so dass diese sitzungsübergreifend zur Verfügung stehen. Die Verwaltung der Zentralkomponente (umfassend Einrichten neuer Nutzer, Kliniken, NSB-Banken) kann beispielsweise von einem Servicepersonal vorgenommen werden. Von der NSB-Bank hochgespielte Informationen können im Spendermanagement und dem zentralen NSB-Präparatemanagement verwaltet werden. Die NSB-Daten können nach (beispielsweise modular austauschbaren) Ordnungskriterien vorgeordnet werden (beispielsweise durch ein Data Warehouse Cube), um vorteilhafterweise eine schnelle effiziente Suche auch bei komplexen Mehrfachtransplantationen zu garantieren. In der Matching Komponente können vorteilhafterweise modular unterschiedliche Matchingalgorithmen genutzt werden, um geeignete Lösungen automatisch oder halbautomatisch zu erzeugen. Alle Abläufe und Interaktionen zwischen den involvierten Parteien können durch die Workflowkomponente gesteuert werden. Des Weiteren können alle Transaktionen und Dienstleistungen durch die Abrechnungskomponente erfasst und ausgewertet werden. Vorteilhafterweise können aufbereitete Abrechungsinformationen an ein Buchhaltungssystem beispielsweise zur Rechnungslegung übergeben werden. Die Follow-Up Informationen können zentral verwaltet und an eine externe Zentralstelle übergeben werden, die beispielsweise Follow-Up Statistiken erzeugen und diese regelmäßig wieder übergeben kann.

**Patentansprüche**

1. Computerimplementiertes Verfahren zur Auswahl eines für die allogene Transplantation geeigneten Nabelschnurblutpräparates,

   **dadurch gekennzeichnet, dass**
   das Verfahren umfasst:

   - Datenaufbereitung, umfassend das Erfassen und Eintragen von physikalischen und informationellen Eigenschaften der Nabelschnurblut-Präparate in eine Datenbank, umfassend:

- Name und Kennung der lagernden Nabelschnurblut-Bank (NSB-Bank),
- Status der lagernden NSB-Bank hinsichtlich internationaler Zertifizierungen,
- Abwicklungszuverlässigkeit der NSB-Bank nach Klassifizierung,
- Ansprechpartner an der betreffenden Bank mit Kontaktdaten,
- Identifikationsnummer des Präparates,
- Anamnese der Mutter, des Kindes und der Familie gemäß Anamnesebogen der Geburtsklinik,
- Ethnie der Mutter, des Vaters und/oder des Kindes,
- Geschlecht des Kindes,
- Einlagerungszeitpunkt des Präparates,
- Informationen zur Aufarbeitung des Präparates,
- Blutgruppe des Präparates,
- HLA-Typus des Präparates,
- Zellzahl (TNC) des Präparates,
- Zellzahl (CD34+) des Präparates,
- Virusstatus des Präparates und/oder
- allele Ausprägungen des Präparates,

- Voreinstellung von Ordnungskriterien, umfassend:

- Name und Kennung der Klinik bzw. des Transplantcenters,
- Name des Koordinators und des behandelnden Arztes mit Kontaktdaten,
- Status der Klinik hinsichtlich internationaler Zertifizierungen (z.B. Fact),
- Durchschnittliche Anzahl an NSB-Transplantationen an der anfragenden Klinik in den letzten drei Jahren,
- Name des Patienten, Versicherungsnummer und weitere Abrechnungsdaten,
- Anamnese des Patienten,
- Indikation und Therapievorschlag des behandelnden Arztes,
- Dringlichkeit nach definierter Klassifizierung,
- HLA-Typus des Patienten,
- Blutgruppe des Patienten,
- Gewicht des Patienten,
- Ethnie des Patienten,
- Geschlecht des Patienten,
- Alter des Patienten,
- Bekannte Allele Ausprägungen des Patienten und/oder Daten einer DNA-Typisierung und/oder
- Erst- oder Wiederholungsbehandlung,

- Patientenrecherche, wobei die Patientenrecherche eine Ermittlung von zum Patienten kompatiblen Präparaten entsprechend der Ordnungskriterien umfasst, wobei die Daten eines Patienten mit den in einer NSB-Datenbank gespeicherten Daten des Präparates verglichen werden und Auskunft gegeben wird, ob das Präparat verträglich für den Patienten ist,
- Bestellung eines Nabelschnurblutpräparates und
- Nachverfolgung, umfassend die Erfassung und Eintragung des Transplantationsverlaufes in der NSB-Datenbank, ausgewählt aus der Gruppe umfassend Anwachsen des Transplantates, Dauer bis zur Funktionsfähigkeit des Transplantates und/oder mögliche Abstoßungsreaktionen,

wobei
die potentiellen Nabelschnur-Präparate nach einem HLA-Match, einem Patientengewicht, einer Anzahl der kernhaltigen Zellen (TNC) und einer Anzahl der hämatopoetischen Zellen (CD34+) geordnet und ausgewählt werden,
wobei
Präparate, bei denen weniger als 75% der CD34+ Zellen in einem Colony Assay Experiment überlebten oder aktiviert wurden, ausgeschlossen werden,
wobei
die Ordnung der potentiellen Nabelschnur-Präparate wie folgt festgelegt ist:

- Erstellung einer Long List von Präparaten durch ein Matchlevel ($ML_{Präp}$) entsprechend der HLA Übereinstimmungen zwischen Präparat und Patient und Blutgruppenverträglichkeit, wobei

$$ML_{Präp} = \begin{cases} 6: HLA_{Präp} \text{ und } HLA_{Pat} \text{ matchen in 6 von 6 Werten und Blutgruppenverträglichkeit} \\ 5: HLA_{Präp} \text{ und } HLA_{Pat} \text{ matchen in 5 von 6 Werten und Blutgruppenverträglichkeit} \\ 4: HLA_{Präp} \text{ und } HLA_{Pat} \text{ matchen in 4 von 6 Werten und Blutgruppenverträglichkeit} \end{cases}$$

wobei Präparate, in welchen weniger als 4 von 6 HLA Typisierungen übereinstimmen, von der Long List ausgeschlossen werden,
- Erstellung einer Short List ($SL_{Single}$), wobei

$$SL_{Single} := \left\{ p \in Präp \left| \frac{OZ_{präp}}{KG_{Pat}} \geq \frac{1}{kg} \wedge ML_{Präp} \geq 4 \right. \right\}$$

$$OZ_{Präp} := \frac{TNC_{Präp}}{ZF_{Präp}}$$

$$ZF_{Präp} := \begin{cases} 3 \times 10^7 : ML_{Präp} = 6 \\ 4 \times 10^7 : ML_{Präp} = 5 \\ 5 \times 10^7 : ML_{Präp} = 4 \end{cases}$$

und die Ordnung der Präparate in der Short List nach Ordnung 1 oder Ordnung 2 erfolgt, wobei

$$\text{Ordnung 1 (SL)} := \left\{ p1 \in SL, p2 \in SL \left| \begin{array}{l} \text{entweder } ML_{p1} > ML_{p2} \\ \text{oder } ML_{p1} = ML_{p2} \wedge OZ_{p1} > OZ_{p2} \\ \text{oder } ML_{p1} = ML_{p2} \wedge OZ_{p1} = OZ_{p2} \wedge CD34_{p1} \geq CD34_{p2} \end{array} \right. \right\}$$

$$\text{Ordnung 2 (SL)} := \left\{ p1 \in SL, p2 \in SL \left| \begin{array}{l} \text{entweder } OZ_{p1} > OZ_{p2} \\ \text{oder } OZ_{p1} = OZ_{p2} \wedge ML_{p1} > ML_{p2} \\ \text{oder } OZ_{p1} = OZ_{p2} \wedge ML_{p1} = ML_{p2} \wedge CD34_{p1}^+ \geq CD34_{p2}^+ \end{array} \right. \right\}$$

wobei

Präp = Nabelschnurblutpräparat
Pat = Patient
$HLA_{Pat}$ = HLA Werte des Patienten
$HLA_{Präp}$ = HLA Werte eines Präparates
$TNC_{Präp}$ = Anzahl der kernbehafteten Zellen eines Präparates
$KG_{Pat}$ = Körpergewicht des Patienten in kg
$CD34+_{Präp}$ = Anzahl der $CD34^+$ Zellen eines Präparates
$ML_{Präp}$ = Matchlevel entsprechend der HLA Übereinstimmung zwischen Präparat und Patient
$ZF_{Präp}$ = der Zellfaktor definiert die benötigten Zellzahl pro kg Patientengewicht bei entsprechndem Matchlevel
$OZ_{Präp}$ = Ordungszahl eines Präparates mit der die Präparate entsprechend TNC und Matchlevel geordnet werden können
$SL_{Single}$ = Shortlist der zu berücksichtigenden Präparate für Einzeltransplantate
Ordung 1 = Reihung zuerst nach Matchlevel, dann nach Ordnungszahl, dann nach $CD34^+$

Ordung 2 = Reihung zuerst nach Ordnungszahl, dann nach Matchlevel, dann nach CD34$^+$.

2. Computerimplementiertes Verfahren nach Anspruch 1,
   **dadurch gekennzeichnet, dass**
   folgende Ordnungskriterien und/oder Ausschlusskriterien herangezogen werden:

   - Präparate mit einer CD34+ Zellzahl höher 10% der TNC Zellzahl,
   - Ausschluß von Präparaten, bei denen im CA (Colony Assay) weniger als 75% der CD34+ Zellen überlebten bzw. aktiviert wurden,
   - Blutgruppenidentität,
   - Ethnische Identität,
   - Geschlecht,
   - Alter des Präparates,
   - Akkreditierungsstandard und/oder
   - Ranking der NSB-Bank.

3. Computerimplementiertes Verfahren nach einem der vorhergehenden Ansprüche,
   **dadurch gekennzeichnet, dass**
   das Verfahren zur Vermittlung von Doppel- oder Mehrfachtransplantationen (Multicord) eingesetzt wird.

4. Computerimplementiertes Verfahren nach dem vorhergehenden Anspruch,
   **dadurch gekennzeichnet, dass**
   die Auswahl der Multicord-Präparate nach folgenden Ordnungskriterien erfolgt:

$$\text{ML}_{P1P2} = \text{Verträglichkeit von 2 Präparaten untereinander:}$$

$$\text{ML}_{P1P2} := \begin{cases} 6 : HLA_{\text{Präp1}} \ und \ HLA_{\text{Präp2}} \ matchen \ in \ 6 \ von \ 6 \ Werten \ und \ Blutgruppenverträglichkeit \\ 5 : HLA_{\text{Präp1}} \ und \ HLA_{\text{Präp2}} \ matchen \ in \ 5 \ von \ 6 \ Werten \ und \ Blutgruppenverträglichkeit \\ 4 : HLA_{\text{Präp1}} \ und \ HLA_{\text{Präp2}} \ matchen \ in \ 4 \ von \ 6 \ Werten \ und \ Blutgruppenverträglichkeit \\ \text{Pr}\ddot{a}parat \ wird \ nicht \ ber\ddot{u}cksichtigt : sonst \end{cases}$$

$$\text{GL}_{Multi} = \text{Grundliste zur Ermittlung der Auswahlliste für Multipäparate}$$

$$\text{GL}_{Multi} := \left\{ p \in \text{Pr}\ddot{a}p \left| \frac{OZ_p}{KG_{Pat}} < \frac{1}{kg} \wedge ML_{P1P2} \geq 4 \right. \right\}$$

$$\text{SL}_{Multi} = \text{Shortlist der zu berücksichtigenden Präparate für Multitransplantate}$$

$$\text{SL}_{Multi} := \left\{ p1 \in GL_{Multi}, p2 \in GL_{Multi} \left| ML_{p1p2} \geq 4 \wedge \frac{OZ_{p1} + OZ_{p2}}{KG_{Pat}} \geq 1 \right. \right\}.$$

5. Computerimplementiertes Verfahren nach einem oder mehreren der vorhergehenden Ansprüche,
   **dadurch gekennzeichnet, dass**
   das Verfahren die Koordination zwischen Klinik, Transplantationszentrum und behandelndem Arzt umfasst.

6. Computerimplementiertes Verfahren nach einem oder mehreren der vorhergehenden Ansprüche,
   **dadurch gekennzeichnet, dass**

das Verfahren für die automatische und vollständige Auswahl für Singlecordoder Multicord-Transplantate verwendet wird.

**7.** Computerimplementiertes Verfahren nach einem oder mehreren der vorhergehenden Ansprüche, **dadurch gekennzeichnet, dass** das Verfahren für die Darstellung der über die Suchkriterien erlangten Ergebnisse eine Matrix verwendet und die Ergebnisse visuell darstellt.

**8.** Computerimplementiertes Verfahren nach einem oder mehreren der vorhergehenden Ansprüche, **dadurch gekennzeichnet, dass** das Verfahren für die Beurteilung des Status der Ordnungskriterienermittlung mittels Statistiken über die zu erwartenden Kosten und die zu benötigende Zeit verwendet wird.

**Claims**

**1.** Computer implemented method for the selection of a cord blood preparation suitable for allogeneic transplantation, **characterized in that** the method comprises:

   - data preparation, comprising the collection and entering of physical and informational properties of the cord blood preparations in a data bank, comprising:

      - Name and identification of the storage cord blood bank (CB-Bank),
      - Status of the storage CB-Bank with regard to international certifications,
      - Processing reliability of the CB-Bank according to classification,
      - Contact person at said bank with contact information,
      - Identification number of the preparation,
      - Anamnesis of the mother, the child and the family according to the anamnesis file of the birth clinic,
      - Ethnicity of the mother, the father and/or the child,
      - Gender of the child ,
      - Storage time point of the preparation,
      - Information regarding maintenance of the preparation,
      - Blood group of the preparation,
      - HLA-Type of the preparation,
      - Number of cells (TNC) of the preparation,
      - Number of cells (CD34+) of the preparation,
      - Viral status of the preparation and/or
      - Allelic characteristics of the preparation,

   - Pre-setting of ordering criteria, comprising:

      - Name and identification of the clinic or transplantation centre,
      - Name of the coordinator and physician responsible for treatment with contact information,
      - Status of the clinic with regard to international certifications (e.g. Fact)
      - Average number of CB-transplantations at requesting clinic in the last three years,
      - Name of the patient, insurance number and additional billing information,
      - Anamnesis of the patient,
      - Indication and proposed therapy from the physician responsible for treatment,
      - Urgency according to defined classification,
      - HLA-Type of the patient,
      - Blood group of the patient,
      - Weight of the patient,
      - Ethnicity of the patient,
      - Gender of the patient,
      - Age of the patient,
      - Known allelic characteristics of the patient and/or data regarding DNA-typing and/or
      - First or repeated treatment,

- Patient search, whereby the patient search comprises determination of preparations compatible with the patient according to the ordering criteria, whereby the data of the patient is compared to the data of the preparation saved in a CB-Databank and a result is provided, whether the preparation is compatible for the patient,
- Ordering of a cord blood preparation and
- Follow up, comprising the recording and entering of the transplantation process in a CB-Database, selected from the group comprising growth of the transplant, duration until functionality of the transplant and/or possible rejection reactions,

whereby
the potential cord blood preparations are ordered and selected according to an HLA-Match, patient weight, the number of nucleated cells (TNC) and the number of haematopoetic cells (CD34+),
whereby
preparations, in which less than 75 % of the CD34+ cells survived or were activated in a Colony Assay Experiment are excluded,
whereby
the ordering of potential cord blood preparations is determined as follows:

- Determination of a Long List of preparations through a Matchlevel ($ML_{prep}$) corresponding to the HLA concordances between preparation and patient and blood group compatibility, whereby

$$\mathrm{ML}_{prep} = \begin{cases} 6 : HLA_{\mathrm{Pr}ep} \text{ and } HLA_{Pat} \text{ match in } 6 \text{ of } 6 \text{ values and blood group compatibility} \\ 5 : HLA_{\mathrm{Pr}ep} \text{ and } HLA_{Pat} \text{ match in } 5 \text{ of } 6 \text{ values and blood group compatibility} \\ 4 : HLA_{\mathrm{Pr}ep} \text{ and } HLA_{Pat} \text{ match in } 4 \text{ of } 6 \text{ values and blood group compatibility} \end{cases}$$

whereby preparations, in which less than 4 of 6 HLA typings concord, are excluded from the Long List,

- Determination of a Short List ($SL_{Single}$), whereby

$$\mathrm{SL}_{Single} := \left\{ p \in \mathrm{Pr}\,ep \left| \frac{OZ_{prep}}{KG_{Pat}} \geq \frac{1}{kg} \wedge ML_{\mathrm{Pr}ep} \geq 4 \right. \right\}$$

$$\mathrm{OZ}_{Prep} := \frac{TNC_{\mathrm{Pr}ep}}{ZF_{\mathrm{Pr}ep}}$$

$$\mathrm{ZF}_{Prep} := \begin{cases} 3 \times 10^7 : ML_{\mathrm{Pr}ep} = 6 \\ 4 \times 10^7 : ML_{\mathrm{Pr}ep} = 5 \\ 5 \times 10^7 : ML_{\mathrm{Pr}ep} = 4 \end{cases}$$

and the ordering of the preparations in the Short List occurs according to Rule 1 or Rule 2, whereby

$$\text{Rule 1 (SL)} := \begin{cases} p1 \in SL, p2 \in SL \left| \begin{array}{l} either\ ML_{p1} > ML_{p2} \\ or\ ML_{p1} = ML_{p2} \wedge OZ_{p1} > OZ_{p2} \\ or\ ML_{p1} = ML_{p2} \wedge OZ_{p1} = OZ_{p2} \wedge CD34_{p1} \geq CD34_{p2} \end{array} \right. \end{cases}$$

$$\text{Rule 2 (SL)} := \left\{ p1 \in SL, p2 \in SL \left| \begin{array}{l} either\ OZ_{p1} > OZ_{p2} \\ or\ OZ_{p1} = OZ_{p2} \wedge ML_{p1} > ML_{p2} \\ or\ OZ_{p1} = OZ_{p2} \wedge ML_{p1} = ML_{p2} \wedge CD34^{+}_{p1} \geq CD34^{+}_{p2} \end{array} \right. \right.$$

whereby

Prep = Cord blood preparation
Pat = Patient
$HLA_{pat}$ = HLA values of the patient
$HLA_{prep}$ = HLA values of the preparation
$TNC_{prep}$ = Number of nucleated cells of the preparation
$KG_{pat}$ = Body weight of the patient in kg
$CD34+_{prep}$ = Number of CD34+ cells of the preparation
$ML_{prep}$ = Match level corresponding to the HLA concordance between preparation and patient
$ZF_{prep}$ = The cell factor defines the necessary number of cells per kg of patient weight for the corresponding match level
$OZ_{prep}$ = ordering number of a preparation, with which the preparation can be ordered according to TNC and match level
$SL_{single}$ = Shortlist of the preparations to be considered for a single transplantation
Rule 1 = sequence according first to match level, then to ordering number, then to CD34+.
Rule 2 = sequence according first to ordering number, then to match level, then to CD34+.

2. Computer implemented method according to claim 1
**characterized in that**
the following ordering criteria and/or exclusion criteria are applied:

- Preparations with a CD34+ cell number of 10 % greater than the number of TNC cells,
- Exclusion of preparations, in which less than 75 % of the CD34+ cells survived or were activated in a CA (Colony Assay),
- Blood group identity,
- Ethnic identity,
- Gender,
- Age of the preparation,
- Accreditation standard and/or
- Ranking of the CB-Bank.

3. Computer implemented method according to one of the preceding claims,
**characterized in that**
the method is applied for the provision of double or multiple transplantations (multi cord).

4. Computer implemented method according to the preceding claim,
**characterized in that**

$ML_{P1P2}$ = Compatibility of two preparations with one another:

$$ML_{P1P2} := \left\{ \begin{array}{l} 6 : HLA_{Prep1}\ and\ HLA_{Prep2}\ matches\ in\ 6\ of\ 6\ values\ and\ blood\ group\ compatibility \\ 5 : HLA_{Prep1}\ and\ HLA_{Prep2}\ matches\ in\ 5\ of\ 6\ values\ and\ blood\ group\ compatibility \\ 4 : HLA_{Prep1}\ and\ HLA_{Prep2}\ matches\ in\ 4\ of\ 6\ values\ and\ blood\ group\ compatibility \\ \Pr eparation\ is\ not\ considered : others \end{array} \right.$$

$GL_{multi}$ = Basic list for determination of the selection list for multiple preparations

$$GL_{Multi} := \left\{ p \in \Pr ep \left| \frac{OZ_p}{KG_{Pat}} < \frac{1}{kg} \wedge ML_{P1P2} \geq 4 \right. \right\}$$

SL$_{multi}$ = Short list of the preparations to be considered for multi transplantations

$$SL_{Multi} := \left\{ p1 \in GL_{Multi}, p2 \in GL_{Multi} \left| ML_{p1p2} \geq 4 \wedge \frac{OZ_{p1} + OZ_{p2}}{KG_{Pat}} \geq 1 \right. \right\}.$$

5. Computer implemented method according to one or more of the preceding claims, **characterized in that** the method comprises coordination between clinic, transplantation centre and physician responsible for treatment.

6. Computer implemented method according to one or more of the preceding claims, **characterized in that** the method is used for the automatic and complete selection of single cord or multi cord transplantations.

7. Computer implemented method according to one or more of the preceding claims, **characterized in that** the method uses a matrix for representation of the results obtained via the ordering criteria and that the results are represented visually.

8. Computer implemented method according to one or more of the preceding claims, **characterized in that** the method is used for evaluation of the status of the determination of ordering criteria using statistics for the expected costs and the required time.

**Revendications**

1. Procédé mis en oeuvre par ordinateur pour sélectionner une préparation de sang ombilical adaptée à la transplantation allogène **caractérisé en ce que** le procédé comprend :

- préparation de données comprenant la saisie et l'inscription de propriétés physiques et informationnelles des préparations de sang ombilical dans une base de données, comprenant

- nom et identification de la banque des préparations de sang ombilical (banque PSO),
- statut de la banque PSO concernant les certifications internationales,
- fiabilité de la procédure de la banque PSO selon classification,
- interlocuteur de la banque concernée avec données de contact,
- numéro d'identification de la préparation,
- anamnèse de la mère, de l'enfant et de la famille conformément au questionnaire d'anamnèse de la maternité,
- ethnie de la mère, du père et/ou de l'enfant,
- sexe de l'enfant,
- date du stockage de la préparation,
- information sur la réalisation de la préparation,
- groupe sanguin de la préparation,
- type HLA de la préparation,
- nombre de cellules (TNC) de la préparation,
- nombre de cellules (CD34+) de la préparation,

- statut de virus de la préparation et/ou
- caractéristiques allèles de la préparation,

- paramétrage préalable de critères d'ordre, comprenant :

- nom et identification de la clinique ou du centre de transplantation,
- nom du coordinateur et du médecin traitant avec données de contact,
- statut de la clinique concernant les certifications internationales, (p.ex. Fact),
- nombre moyen de transplantations PSO dans la clinique en question ces trois dernières années,
- nom du patient, numéro d'assurance et autres données de décompte,
- anamnèse du patient,
- indication et proposition de thérapie du médecin traitant,
- urgence selon la classification définie,
- type HLA du patient,
- groupe sanguin du patient,
- poids du patient,
- ethnie du patient,
- sexe du patient,
- âge du patient,
- caractéristiques allèles connues du patient et/ou données d'un typage ADN et/ou
- premier traitement ou traitement répété,

- recherche de patient, la recherche de patient comprenant une détermination de préparations compatibles avec le patient conformément aux critères d'ordre, les données d'un patient étant comparées aux données de la préparation enregistrées dans la banque de données PSO et renseignant si la préparation est compatible avec le patient,
- commande d'une préparation de sang ombilical et
- suivi, comprenant la saisie et l'inscription du déroulement de la transplantation dans la banque de données PSO, sélectionnée à partir du groupe comprenant la croissance de la greffe, la durée jusqu'à la fonctionnabilité de la greffe et/ou les possibles réactions de rejet,
les préparations de sang ombilical potentielles sont classées et sélectionnées selon une compatibilité HLA, un poids de patient, un nombre de cellules nuclées (TNC) et un nombre de cellules hématopoïétiques (CD34+),
les préparations dans lesquelles, dans une expérience Colony Assay, moins de 75% des cellules CD34+ ont survécu ou ont été activées, étant exclues,

l'ordre des préparations de sang ombilical potentielles étant déterminé comme suit :

- établissement d'une liste longue de préparation par un niveau de compatibilité (ML$_{Präp}$) conformément à la concordance HLA entre la préparation et le patient et le compatibilité du groupe sanguin,

$$\text{ML}_{\text{Präp}} = \begin{cases} 6 : HLA_{\text{Präp}} \text{ et } HLA_{Pat} \text{ sont compatibles dans 6 valeurs sur 6 et compatibilité de groupe sanguin} \\ 5 : HLA_{\text{Präp}} \text{ et } HLA_{Pat} \text{ sont compatibles dans 5 valeurs sur 6 et compatibilité de groupe sanguin} \\ 4 : HLA_{\text{Präp}} \text{ et } HLA_{Pat} \text{ sont compatibles dans 4 valeurs sur 6 et compatibilité de groupe sanguin} \end{cases}$$

les préparations, dans lesquelles moins de 4 typages HLA sur 6 concordent, sont exclues de la liste longue,
- établissement d'une liste courte(SL$_{Single}$),

$$\text{SL}_{\text{Single}} := \left\{ p \in \text{Pr}\ddot{a}p \left| \frac{OZ_{pr\ddot{a}p}}{KG_{Pat}} \geq \frac{1}{kg} \wedge ML_{\text{Pr}\ddot{a}p} \geq 4 \right. \right\}$$

$$\text{OZ}_{\text{Präp}} := \frac{TNC_{\text{Pr}\ddot{a}p}}{ZF_{\text{Pr}\ddot{a}p}}$$

$$ZF_{Pr\ddot{a}p} := \begin{cases} 3 \times 10^7 : ML_{Pr\ddot{a}p} = 6 \\ 4 \times 10^7 : ML_{Pr\ddot{a}p} = 5 \\ 5 \times 10^7 : ML_{Pr\ddot{a}p} = 4 \end{cases}$$

et l'ordre des préparations dans la courte liste s'effectue selon l'ordre 1 ou 2,

$$Ordre\ 1\ (SL) := \begin{cases} p1 \in SL, p2 \in SL \end{cases} \begin{vmatrix} soit\ ML_{p1} > ML_{p2} \\ ou\ ML_{p1} = ML_{p2} \wedge OZ_{p1} > OZ_{p2} \\ ou\ ML_{p1} = ML_{p2} \wedge OZ_{p1} = OZ_{p2} \wedge CD34_{p1} \geq CD34_{p2} \end{vmatrix}$$

$$Ordre\ 2\ (SL) := \begin{cases} p1 \in SL, p2 \in SL \end{cases} \begin{vmatrix} soit\ OZ_{p1} > OZ_{p2} \\ ou\ OZ_{p1} = OZ_{p2} \wedge ML_{p1} > ML_{p2} \\ ou\ OZ_{p1} = OZ_{p2} \wedge ML_{p1} = ML_{p2} \wedge CD34_{p1}^+ \geq CD34_{p2}^+ \end{vmatrix}$$

Präp = préparation de sang ombilical
Pat = patient
$HLA_{Pat}$ = valeurs HLA du patient
$HLA_{Präp}$ = valeurs HLA d'une préparation
$TNC_{Präp}$ = nombre de cellules nuclées d'une préparation
$KG_{Pat}$ = poids du corps du patient en kg
$CD34+_{Präp}$ = nombre des cellules $CD34^+$ d'une préparation
$_{ML}Präp$ = niveau de compatibilité conformément à la concordance HLA entre la préparation et le patient
$ZF_{Präp}$ = Le facteur cellulaire définit le nombre de cellules nécessaires par kg du poids du patient pour le niveau correspondant de compatibilité
$OZ_{Präp}$ = nombre d'ordre d'une préparation avec lequel les préparations peuvent être classées conformément au TNC et au niveau de compatibilité
$SL_{Single}$ = La courte liste des implantations individuelles pour les greffes individuelles à prendre en compte
Ordre 1 = classement d'abord en fonction du niveau de compatibilité, ensuite en fonction du nombre d'ordre, ensuite selon $CD34^+$
Ordre 2 = classement d'abord en fonction du nombre d'ordre, ensuite en fonction du niveau de compatibilité, ensuite selon $CD34^+$

2. Procédé mis en oeuvre par ordinateur selon l'une des revendications précédentes **caractérisé en ce que**
l'on a recours aux critères d'ordre et/ou aux critères d'exclusion suivants :

- préparations avec un nombre de cellules CD34+ supérieur à 10% au nombre de cellules TNC,
- exclusion de préparations où dans le CA (Colony Assay) moins de 75% des cellules CD34+ survivent ou ont été activées,
- identité du groupe sanguin
- identité ethnique,
- sexe,
- âge de la préparation,
- norme d'accréditation et/ou
- classement de la banque PSO.

3. Procédé mis en oeuvre par ordinateur selon l'une des revendications précédentes **caractérisé en ce que**
il est utilisé pour déterminer les transplantations doubles ou multiples (multicord).

4. Procédé mis en oeuvre par ordinateur selon la revendication précédente

**caractérisé en ce que**

la sélection des préparations multicord s'effectue selon les critères d'ordre suivants :

$$ML_{P1P2} = \text{compatibilité de 2 préparations entre elle :}$$

$$ML_{P1P2} := \begin{cases} 6: HLA_{Pr\ddot{a}p} \text{ et } HLA_{Pat} \text{ sont compatibles dans 6 valeurs sur 6 et compatibilité de groupe sanguin} \\ 5: HLA_{Pr\ddot{a}p} \text{ et } HLA_{Pat} \text{ sont compatibles dans 5 valeurs sur 6 et compatibilité de groupe sanguin} \\ 4: HLA_{Pr\ddot{a}p} \text{ et } HLA_{Pat} \text{ sont compatibles dans 4 valeurs sur 6 et compatibilité de groupe sanguin} \\ \Pr\acute{e}paration \, non \, prise \, en \, compte : \sin on \end{cases}$$

$$GL_{Multi} = \text{liste de base pour déterminer la liste de sélection des multipréparations}$$

$$GL_{Multi} := \left\{ p \in \Pr\ddot{a}p \left| \frac{OZ_p}{KG_{Pat}} < \frac{1}{kg} \wedge ML_{P1P2} \geq 4 \right. \right\}$$

$$SL_{Multi} = \text{courte liste des préparations à prendre pour les multigreffes}$$

$$SL_{Multi} := \left\{ p1 \in GL_{Multi}, p2 \in GL_{Multi} \left| ML_{p1p2} \geq 4 \wedge \frac{OZ_{p1} + OZ_{p2}}{KG_{Pat}} \geq 1 \right. \right\}.$$

5. Procédé mis en oeuvre par ordinateur selon un ou plusieurs revendications précédentes **caractérisé en ce que**
   le procédé comprend la coordination entre la clinique, le centre de transplantation et le médecin traitant.

6. Procédé mis en oeuvre par ordinateur selon une ou plusieurs revendications précédentes **caractérisé en ce que**
   le procédé est utilisé pour la sélection automatique et complète pour les greffes siglecord ou multicord.

7. Procédé mis en oeuvre par ordinateur selon une ou plusieurs revendications précédentes **caractérisé en ce que**
   le procédé pour la représentation des résultats obtenus par les critères de recherche utilise une matrice et représente les résultats visuellement.

8. Procédé mis en oeuvre par ordinateur selon une ou plusieurs revendications précédentes **caractérisé en ce que**
   on utilise le procédé pour l'évaluation du statut de la détermination de critères d'ordre via des statistiques sur les coûts à attendre et le temps nécessaire.

**Figur 1:**

Figur 2:

Datenaufbereitung

NSB Präparate Upload: NSB Daten (Unit Reports) werden in NSB Bank erfasst und in das System eingespielt

Erweiterung der NSB Präparat Verträglichkeitsmatrix: Daten aller neuen NSB Präparate werden mit den im System vorhandenen NSB Präparate auf Kompatibilität geprüft und Verträglichkeitsmatrix wird erweitert

Voreinstellung der Suchkriterien

Festlegung der Standard-Suchprofile für Kliniken und Ärzte

Individualisierung der Ordnungs und Ausschlusskriterien nach Koordinatorpräferenzen

Transplantat Suche

Neuer Patient wird erfasst, zusätzlice NSB Präparate können eingespielt, Suchparameter können fallspezifisch angepasst werden

Basic Serach: Ermittlung aller zum Patienten kompatiblen Präparate entsprechend der voreingestellten Ordungs- und Auschlusskriterien (Long List)

Advanced Search

Ermittlung der möglichen Singletransplantate (Shortlist SingleCord)

Ermittlung der möglichen Multitransplantate (Shortlist MultiCord)

Berechung des Budgets und der benötigten Zeit sowie der Ergebnisqualität pro Transplant aufgrund der noch durchzuführenden Tests etc.

Lösungsgenerierung

Absolute Reihung aller Shortlistpräparate nach vorgegebenen Präferenzen

Betätigung / Korrektur des Lösungsvorschlages durch den Arzt

Erzeugung der Patientenakte: Laufzettel, Unit Report, Entscheidungsvorlage, Patientendokumentation

Optional: Comparison View für selektierte Präparate und manuelle Auswahl

Bestellabwicklung & Nachverfolung

Bestellung des NSB Präparates

Reservierung von Präparaten und Festlegung von Backup-Präparaten

Bestellauslösung mit Versand und Fakturierung

Eingangskontrolle der NSB-Präparate

Abwicklung von Verifikationsaufträgen (DNA Proben, High Resolution Typisierung, ...)

Nachverfolgung des Patienten (Follow-Up)

**Figur 3:**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20020132343 A1
- US 20020168639 A1
- GB 2407193 A
- US 20040121369 A1
- WO 02077640 A2
- US 20080014174 A1
- DE 60030978 T2
- WO 2005097190 A2